# EUROPEAN PATENT SPECIFICATION

(11) **EP 0 557 459 B1**
(45) Date of publication and mention of the grant of the patent: **22.10.1997**
(21) Application number: 92902004.8
(22) Date of filing: 12.11.1991
(51) Int. Cl.: C12N 15/65, C12N 15/62, C12N 5/10, A61K 48/00

(54) **BIFUNCTIONAL SELECTABLE FUSION GENES**
BIFUNKTIONELLE WÄHLBARE FUSIONSGENE
GENES DE FUSION SELECTIONNABLES BIFONCTIONNELS

(30) Priority: 13.11.1990 US 612326
(43) Date of publication of application: 01.09.1993
(73) Proprietor: IMMUNEX CORPORATION, Seattle Washington 98101 (US)
(72) Inventor: LUPTON, Stephen, D., Seattle, WA 98115 (US)
(74) Representative: Goldin, Douglas Michael
(86) International application number: US9108442
(87) International publication number: WO9208796

(56) References cited:
- EP-A- 293 193
- EP-A- 415 731
- THE EMBO JOURNAL, vol. 6, no. 1, 1987; pp. 187-193
- MOLECULAR & CELLULAR BIOLOGY, vol. 6, no. 3, 1986; pp. 792-800
- JOURNAL OF MOLECULAR BIOLOGY, vol. 150, 1981; F. COLBERE-GARAPIN et al., pp. 1-14
- JOURNAL OF BIOLOGICAL CHEMISTRY, vol. 264, no. 13, 05 May 1989, Baltimore, MD (US); U.A. GERMANN et al., pp. 7418-7424
- NUCLEIC ACIDS RESEARCH, vol. 11, no. 19, 1983, Arlington, VA (US); K.R. KASTER et al., pp. 6895-6911
- CHEMICAL ABSTRACTS, vol. 103, no. 7, 19 August 1985, Columbus, OH (US); C. LASSARE et al., p. 136, AN 49062
- MOLECULAR & CELLULAR BIOLOGY, vol. 11, no. 6, June 1991, Washington, DC (US); S.D. LUPTON et al., pp. 3374-3378

### SEQUENCE LISTING

(1) GENERAL INFORMATION:
   (i) APPLICANT: Lupton, Stephen D.
   (ii) TITLE OF INVENTION: Bifunctional Selectable Fusion Genes
   (iii) NUMBER OF SEQUENCES: 2
   (iv) CORRESPONDENCE ADDRESS:
      (A) ADDRESSEE: Immunex Corporation
      (B) STREET: 51 University Street
      (C) CITY: Seattle
      (D) STATE: WA
      (E) COUNTRY: USA
      (F) ZIP: 98101
   (v) COMPUTER READABLE FORM:
      (A) MEDIUM TYPE: Diskette, 3.50 inch, 800 Kb storage
      (B) COMPUTER: Apple Macintosh
      (C) OPERATING SYSTEM: Maintosh
      (D) SOFTWARE: Microsoft Word
   (vi) CURRENT APPLICATION DATA:
      (A) APPLICATION NUMBER:
      (B) FILING DATE:
      (C) CLASSIFICATION:
   (viii) PRIOR APPLICATION DATA:
      (A) APPLICATION NUMBER: 07/612,326
      (B) FILING DATE: 13-NOV-1990
   (ix) ATTORNEY/AGENT INFORMATION:
      (A) NAME: Wight, Christopher L.
      (B) REGISTRATION NUMBER: 31,680
      (C) REFERENCE/DOCKET NUMBER: 2702-A
   (x) TELECOMMUNICATION INFORMATION:
      (A) TELEPHONE: (206)587-0430
      (B) TELEFAX: (206)587-0606
      (C) TELEX: 756822
(2) INFORMATION FOR SEQ ID NO:1:
   (i) SEQUENCE CHARACTERISTICS:
      (A) LENGTH: 2076 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: N
   (iv) ANTI-SENSE: N
   (ix) FEATURE:
      (A) NAME/KEY: CDS
      (B) LOCATION: 1..2076
      (D) OTHER INFORMATION:
   (ix) FEATURE:
      (A) NAME/KEY: mat_peptide
      (B) LOCATION: 1..2073
      (D) OTHER INFORMATION:
   (xi) SEQUENCE DESCRIPTION: SEQ ID NO:1:
   ATG AAA AAG CCT GAA CTC ACC GCG ACG TCT GTC GAG AAG TTT CTG ATC      48
   Met Lys Lys Pro Glu Leu Thr Ala Thr Ser Val Glu Lys Phe Leu Ile
    1               5                  10                  15
   GAA AAG TTC GAC AGC GTC TCC GAC CTG ATG CAG CTC TCG GAG GGC GAA      96
   Glu Lys Phe Asp Ser Val Ser Asp Leu Met Gln Leu Ser Glu Gly Glu
               20                  25                  30
   GAA TCT CGT GCT TTC AGC TTC GAT GTA GGA GGG CGT GGA TAT GTC CTG     144
   Glu Ser Arg Ala Phe Ser Phe Asp Val Gly Gly Arg Gly Tyr Val Leu
           35                  40                  45
   CGG GTA AAT AGC TGC GCC GAT GGT TTC TAC AAA GAT CGT TAT GTT TAT     192
   Arg Val Asn Ser Cys Ala Asp Gly Phe Tyr Lys Asp Arg Tyr Val Tyr
       50                  55                  60
   CGG CAC TTT GCA TCG GCC GCG CTC CCG ATT CCG GAA GTG CTT GAC ATT     240
   Arg His Phe Ala Ser Ala Ala Leu Pro Ile Pro Glu Val Leu Asp Ile
   65                  70                  75                  80
   GGG GAA TTC AGC GAG AGC CTG ACC TAT TGC ATC TCC CGC CGT GCA CAG     288
   Gly Glu Phe Ser Glu Ser Leu Thr Tyr Cys Ile Ser Arg Arg Ala Gln
                   85                  90                  95
   GGT GTC ACG TTG CAA GAC CTG CCT GAA ACC GAA CTG CCC GCT GTT CTG     336
   Gly Val Thr Leu Gln Asp Leu Pro Glu Thr Glu Leu Pro Ala Val Leu
               100                 105                 110
   CAG CCG GTC GCG GAG GCC ATG GAT GCG ATC GCT GCG GCC GAT CTT AGC     384
   Gln Pro Val Ala Glu Ala Met Asp Ala Ile Ala Ala Ala Asp Leu Ser
           115                 120                 125
   CAG ACG AGC GGG TTC GGC CCA TTC GGA CCG CAA GGA ATC GGT CAA TAC     432
   Gln Thr Ser Gly Phe Gly Pro Phe Gly Pro Gln Gly Ile Gly Gln Tyr
       130                 135                 140
   ACT ACA TGG CGT GAT TTC ATA TGC GCG ATT GCT GAT CCC CAT GTG TAT    480
   Thr Thr Trp Arg Asp Phe Ile Cys Ala Ile Ala Asp Pro His Val Tyr
   145                 150                 155                 160
   CAC TGG CAA ACT GTG ATG GAC GAC ACC GTC AGT GCG TCC GTC GCG CAG    528
   His Trp Gln Thr Val Met Asp Asp Thr Val Ser Ala Ser Val Ala Gln
                   165                 170                 175
   GCT CTC GAT GAG CTG ATG CTT TGG GCC GAG GAC TGC CCC GAA GTC CGG    576
   Ala Leu Asp Glu Leu Met Leu Trp Ala Glu Asp Cys Pro Glu Val Arg
               180                 185                 190
   CAC CTC GTG CAC GCG GAT TTC GGC TCC AAC AAT GTC CTG ACG GAC AAT    624
   His Leu Val His Ala Asp Phe Gly Ser Asn Asn Val Leu Thr Asp Asn
           195                 200                 205
   GGC CGC ATA ACA GCG GTC ATT GAC TGG AGC GAG GCG ATG TTC GGG GAT    672
   Gly Arg Ile Thr Ala Val Ile Asp Trp Ser Glu Ala Met Phe Gly Asp
       210                 215                 220
   TCC CAA TAC GAG GTC GCC AAC ATC TTC TTC TGG AGG CCG TGG TTG GCT    720
   Ser Gln Tyr Glu Val Ala Asn Ile Phe Phe Trp Arg Pro Trp Leu Ala
   225                 230                 235                 240
   TGT ATG GAG CAG CAG ACG CGC TAC TTC GAG CGG AGG CAT CCG GAG CTT    768
   Cys Met Glu Gln Gln Thr Arg Tyr Phe Glu Arg Arg His Pro Glu Leu
                   245                 250                 255
   GCA GGA TCG CCG CGG CTC CGG GCG TAT ATG CTC CGC ATT GGT CTT GAC    816
   Ala Gly Ser Pro Arg Leu Arg Ala Tyr Met Leu Arg Ile Gly Leu Asp
               260                 265                 270
   CAA CTC TAT CAG AGC TTG GTT GAC GGC AAT TTC GAT GAT GCA GCT TGG    864
   Gln Leu Tyr Gln Ser Leu Val Asp Gly Asn Phe Asp Asp Ala Ala Trp
           275                 280                 285
   GCG CAG GGT CGA TGC GAC GCA ATC GTC CGA TCC GGA GCC GGG ACT GTC    912
   Ala Gln Gly Arg Cys Asp Ala Ile Val Arg Ser Gly Ala Gly Thr Val
       290                 295                 300
   GGG CGT ACA CAA ATC GCC CGC AGA AGC GCG GCC GTC TGG ACC GAT GGC    960
   Gly Arg Thr Gln Ile Ala Arg Arg Ser Ala Ala Val Trp Thr Asp Gly
   305                 310                 315                 320
   TGT GTA GAA GTC GCG TCT GCG TTC GAC CAG GCT GCG CGT TCT CGC GGC   1008
   Cys Val Glu Val Ala Ser Ala Phe Asp Gln Ala Ala Arg Ser Arg Gly
                   325                 330                 335
   CAT AGC AAC CGA CGT ACG GCG TTG CGC CCT CGC CGG CAG CAA GAA GCC   1056
   His Ser Asn Arg Arg Thr Ala Leu Arg Pro Arg Arg Gln Gln Glu Ala
               340                 345                 350
   ACG GAA GTC CGC CCG GAG CAG AAA ATG CCC ACG CTA CTG CGG GTT TAT   1104
   Thr Glu Val Arg Pro Glu Gln Lys Met Pro Thr Leu Leu Arg Val Tyr
           355                 360                 365
   ATA GAC GGT CCC CAC GGG ATG GGG AAA ACC ACC ACC ACG CAA CTG CTG   1152
   Ile Asp Gly Pro His Gly Met Gly Lys Thr Thr Thr Thr Gln Leu Leu
       370                 375                 380
   GTG GCC CTG GGT TCG CGC GAC GAT ATC GTC TAC GTA CCC GAG CCG ATG   1200
   Val Ala Leu Gly Ser Arg Asp Asp Ile Val Tyr Val Pro Glu Pro Met
   385                 390                 395                 400
   ACT TAC TGG CGG GTG CTG GGG GCT TCC GAG ACA ATC GCG AAC ATC TAC   1248
   Thr Tyr Trp Arg Val Leu Gly Ala Ser Glu Thr Ile Ala Asn Ile Tyr
                   405                 410                 415
   ACC ACA CAA CAC CGC CTC GAC CAG GGT GAG ATA TCG GCC GGG GAC GCG   1296
   Thr Thr Gln His Arg Leu Asp Gln Gly Glu Ile Ser Ala Gly Asp Ala
               420                 425                 430
   GCG GTG GTA ATG ACA AGC GCC CAG ATA ACA ATG GGC ATG CCT TAT GCC   1344
   Ala Val Val Met Thr Ser Ala Gln Ile Thr Met Gly Met Pro Tyr Ala
           435                 440                 445
   GTG ACC GAC GCC GTT CTG GCT CCT CAT ATC GGG GGG GAG GCT GGG AGC   1392
   Val Thr Asp Ala Val Leu Ala Pro His Ile Gly Gly Glu Ala Gly Ser
       450                 455                 460
   TCA CAT GCC CCG CCC CCG GCC CTC ACC CTC ATC TTC GAC CGC CAT CCC    1440
   Ser His Ala Pro Pro Pro Ala Leu Thr Leu Ile Phe Asp Arg His Pro
   465                 470                 475                 480
   ATC GCC GCC CTC CTG TGC TAC CCG GCC GCG CGG TAC CTT ATG GGC AGC    1488
   Ile Ala Ala Leu Leu Cys Tyr Pro Ala Ala Arg Tyr Leu Met Gly Ser
                   485                 490                 495
   ATG ACC CCC CAG GCC GTG CTG GCG TTC GTG GCC CTC ATC CCG CCG ACC    1536
   Met Thr Pro Gln Ala Val Leu Ala Phe Val Ala Leu Ile Pro Pro Thr
               500                 505                 510
   TTG CCC GGC ACC AAC ATC GTG CTT GGG GCC CTT CCG GAG GAC AGA CAC    1584
   Leu Pro Gly Thr Asn Ile Val Leu Gly Ala Leu Pro Glu Asp Arg His
           515                 520                 525
   ATC GAC CGC CTG GCC AAA CGC CAG CGC CCC GGC GAG CGG CTG GAC CTG    1632
   Ile Asp Arg Leu Ala Lys Arg Gln Arg Pro Gly Glu Arg Leu Asp Leu
       530                 535                 540
   GCT ATG CTG GCT GCG ATT CGC CGC GTT TAC GGG CTA CTT GCC AAT ACG    1680
   Ala Met Leu Ala Ala Ile Arg Arg Val Tyr Gly Leu Leu Ala Asn Thr
   545                 550                 555                 560
   GTG CGG TAT CTG CAG GGC TGC GGG TCG TGG CGG GAG GAC TGG GGA CAG    1728
   Val Arg Tyr Leu Gln Gly Cys Gly Ser Trp Arg Glu Asp Trp Gly Gln
                   565                 570                 575
   CTT TCG GGG ACG GCC GTG CCG CCC CAG GGT GCC GAG CCC CAG AGC AAC    1776
   Leu Ser Gly Thr Ala Val Pro Pro Gln Gly Ala Glu Pro Gln Ser Asn
               580                 585                 590
   GCG GGC CCA CGA CCC CAT ATC GGG GAC ACG TTA TTT ACC CTG TTT CGG    1824
   Ala Gly Pro Arg Pro His Ile Gly Asp Thr Leu Phe Thr Leu Phe Arg
           595                 600                 605
   GCC CCC GAG TTG CTG GCC CCC AAC GGC GAC CTG TAT AAC GTG TTT GCC    1872
   Ala Pro Glu Leu Leu Ala Pro Asn Gly Asp Leu Tyr Asn Val Phe Ala
       610                 615                 620
   TGG GCC TTG GAC GTC TTG GCC AAA CGC CTC CGT TCC ATG CAC GTC TTT    1920
   Trp Ala Leu Asp Val Leu Ala Lys Arg Leu Arg Ser Met His Val Phe
   625                 630                 635                 640
   ATC CTG GAT TAC GAC CAA TCG CCC GCC GGC TGC CGG GAC GCC CTG CTG   1968
   Ile Leu Asp Tyr Asp Gln Ser Pro Ala Gly Cys Arg Asp Ala Leu Leu
                   645                 650                 655
   CAA CTT ACC TCC GGG ATG GTC CAG ACC CAC GTC ACC ACC CCC GGC TCC   2016
   Gln Leu Thr Ser Gly Met Val Gln Thr His Val Thr Thr Pro Gly Ser
               660                 665                 670
   ATA CCG ACG ATA TGC GAC CTG GCG CGC ACG TTT GCC CGG GAG ATG GGG   2064
   Ile Pro Thr Ile Cys Asp Leu Ala Arg Thr Phe Ala Arg Glu Met Gly
           675                 680                 685
   GAG GCT AAC TGA                                                   2076
   Glu Ala Asn
       690
(2) INFORMATION FOR SEQ ID NO:2:
   (i) SEQUENCE CHARACTERISTICS:
      (A) LENGTH: 691 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Lys Pro Glu Leu Thr Ala Thr Ser Val Glu Lys Phe Leu Ile
    1               5                  10                  15
   Glu Lys Phe Asp Ser Val Ser Asp Leu Met Gln Leu Ser Glu Gly Glu
               20                  25                  30
   Glu Ser Arg Ala Phe Ser Phe Asp Val Gly Gly Arg Gly Tyr Val Leu
           35                  40                  45
   Arg Val Asn Ser Cys Ala Asp Gly Phe Tyr Lys Asp Arg Tyr Val Tyr
       50                  55                  60
   Arg His Phe Ala Ser Ala Ala Leu Pro Ile Pro Glu Val Leu Asp Ile
   65                  70                  75                  80
   Gly Glu Phe Ser Glu Ser Leu Thr Tyr Cys Ile Ser Arg Arg Ala Gln
                   85                  90                  95
   Gly Val Thr Leu Gln Asp Leu Pro Glu Thr Glu Leu Pro Ala Val Leu
               100                 105                 110
   Gln Pro Val Ala Glu Ala Met Asp Ala Ile Ala Ala Ala Asp Leu Ser
           115                 120                 125
   Gln Thr Ser Gly Phe Gly Pro Phe Gly Pro Gln Gly Ile Gly Gln Tyr
       130                 135                 140
   Thr Thr Trp Arg Asp Phe Ile Cys Ala Ile Ala Asp Pro His Val Tyr
   145                 150                 155                 160
   His Trp Gln Thr Val Met Asp Asp Thr Val Ser Ala Ser Val Ala Gln
                   165                 170                 175
   Ala Leu Asp Glu Leu Met Leu Trp Ala Glu Asp Cys Pro Glu Val Arg
               180                 185                 190
   His Leu Val His Ala Asp Phe Gly Ser Asn Asn Val Leu Thr Asp Asn
           195                 200                 205
   Gly Arg Ile Thr Ala Val Ile Asp Trp Ser Glu Ala Met Phe Gly Asp
       210                 215                 220
   Ser Gln Tyr Glu Val Ala Asn Ile Phe Phe Trp Arg Pro Trp Leu Ala
   225                 230                 235                 240
   Cys Met Glu Gln Gln Thr Arg Tyr Phe Glu Arg Arg His Pro Glu Leu
                   245                 250                 255
   Ala Gly Ser Pro Arg Leu Arg Ala Tyr Met Leu Arg Ile Gly Leu Asp
               260                 265                 270
   Gln Leu Tyr Gln Ser Leu Val Asp Gly Asn Phe Asp Asp Ala Ala Trp
           275                 280                 285
   Ala Gln Gly Arg Cys Asp Ala Ile Val Arg Ser Gly Ala Gly Thr Val
       290                 295                 300
   Gly Arg Thr Gln Ile Ala Arg Arg Ser Ala Ala Val Trp Thr Asp Gly
   305                 310                 315                 320
   Cys Val Glu Val Ala Ser Ala Phe Asp Gln Ala Ala Arg Ser Arg Gly
                   325                 330                 335
   His Ser Asn Arg Arg Thr Ala Leu Arg Pro Arg Arg Gln Gln Glu Ala
               340                 345                 350
   Thr Glu Val Arg Pro Glu Gln Lys Met Pro Thr Leu Leu Arg Val Tyr
           355                 360                 365
   Ile Asp Gly Pro His Gly Met Gly Lys Thr Thr Thr Thr Gln Leu Leu
       370                 375                 380
   Val Ala Leu Gly Ser Arg Asp Asp Ile Val Tyr Val Pro Glu Pro Met
   385                 390                 395                 400
   Thr Tyr Trp Arg Val Leu Gly Ala Ser Glu Thr Ile Ala Asn Ile Tyr
                   405                 410                 415
   Thr Thr Gln His Arg Leu Asp Gln Gly Glu Ile Ser Ala Gly Asp Ala
               420                 425                 430
   Ala Val Val Met Thr Ser Ala Gln Ile Thr Met Gly Met Pro Tyr Ala
           435                 440                 445
   Val Thr Asp Ala Val Leu Ala Pro His Ile Gly Gly Glu Ala Gly Ser
       450                 455                 460
   Ser His Ala Pro Pro Pro Ala Leu Thr Leu Ile Phe Asp Arg His Pro
   465                 470                 475                 480
   Ile Ala Ala Leu Leu Cys Tyr Pro Ala Ala Arg Tyr Leu Met Gly Ser
                   485                 490                 495
   Met Thr Pro Gln Ala Val Leu Ala Phe Val Ala Leu Ile Pro Pro Thr
               500                 505                 510
   Leu Pro Gly Thr Asn Ile Val Leu Gly Ala Leu Pro Glu Asp Arg His
           515                 520                 525
   Ile Asp Arg Leu Ala Lys Arg Gln Arg Pro Gly Glu Arg Leu Asp Leu
       530                 535                 540
   Ala Met Leu Ala Ala Ile Arg Arg Val Tyr Gly Leu Leu Ala Asn Thr
   545                 550                 555                 560
   Val Arg Tyr Leu Gln Cys Gly Gly Ser Trp Arg Glu Asp Trp Gly Gln
                   565                 570                 575
   Leu Ser Gly Thr Ala Val Pro Pro Gln Gly Ala Glu Pro Gln Ser Asn
               580                 585                 590
   Ala Gly Pro Arg Pro His Ile Gly Asp Thr Leu Phe Thr Leu Phe Arg
           595                 600                 605
   Ala Pro Glu Leu Leu Ala Pro Asn Gly Asp Leu Tyr Asn Val Phe Ala
       610                 615                 620
   Trp Ala Leu Asp Val Leu Ala Lys Arg Leu Arg Ser Met His Val Phe
   625                 630                 635                 640
   Ile Leu Asp Tyr Asp Gln Ser Pro Ala Gly Cys Arg Asp Ala Leu Leu
                   645                 650                 655
   Gln Leu Thr Ser Gly Met Val Gln Thr His Val Thr Thr Pro Gly Ser
               660                 665                 670
   Ile Pro Thr Ile Cys Asp Leu Ala Arg Thr Phe Ala Arg Glu Met Gly
           675                 680                 685
   Glu Ala Asn
       690

## Description

### BACKGROUND OF THE INVENTION

The present invention relates generally to genes expressing selectable phenotypes. More particularly, the present invention relates to genes capable of co-expressing both dominant positive selectable and negative selectable phenotypes.

Genes which express a selectable phenotype are widely used in recombinant DNA technology as a means for identifying and isolating host cells into which the gene has been introduced. Typically, the gene expressing the selectable phenotype is introduced into the host cell as part of a recombinant expression vector. Positive selectable genes provide a means to identify and/or isolate cells that have retained introduced genes in a stable form, and, in this capacity, have greatly facilitated gene transfer and the analysis of gene function. Negative selectable genes, on the other hand, provide a means for eliminating cells that retain the introduced gene.

A variety of genes are available which confer selectable phenotypes on animal cells. The bacterial neomycin phosphotransferase *(neo)* (Colbere-Garapin et al., *J. Mol. Biol. 150:1,* 1981), hygromycin phosphotransferase *(hph)* (Santerre et al., *Gene 30:147,* 1984), and xanthine-guanine phosphoribosyl transferase (*gpt*) (Mulligan and Berg, *Proc. Natl. Acad. Sci. USA 78:2072,* 1981) genes are widely used dominant positive selectable genes. The *Herpes simplex* virus type I thymidine kinase (HSV-I TK) gene (Wigler et al., *Cell 11*:223, 1977), and the cellular adenine phosphoribosyltransferase (APRT) (Wigler et al., *Proc. Natl. Acad. Sci. USA* 76:1373, 1979) and hypoxanthine phosphoribosyltransferase (HPRT) genes (Jolly et al., *Proc. Natl. Acad. Sci. USA 80*:477, 1983) are commonly used recessive positive selectable genes. In general, dominant selectable genes are more versatile than recessive genes, because the use of recessive genes is limited to mutant cells deficient in the selectable function, whereas dominant genes may be used in wild-type cells.

Several genes confer negative as well as positive selectable phenotypes, including the HSV-I TK, HPRT, APRT and *gpt* genes. These genes encode enzymes which catalyze the conversion of nucleoside or purine analogs to cytotoxic intermediates. The nucleoside analog GCV is an efficient substrate for HSV-I TK, but a poor substrate for cellular TK, and therefore may be used for negative selection against the HSV-I TK gene in wild-type cells (St. Clair et al., *Antimicrob. Agents Chemother. 31:844,* 1987). However, the HSV-I TK gene may only be used effectively for positive selection in mutant cells lacking cellular TK activity. Use of the HPRT and APRT genes for either positive or negative selection is similarly limited to HPRT- or APRT- cells, respectively (Fenwick, "The HGPRT System", pp. 333-373, M. Gottesman (ed.), *Molecular Cell Genetics,* John Wiley and Sons, New York, 1985; Taylor et al., "The APRT System", pp., 311-332, M. Gottesman (ed.), *Molecular Cell Genetics,* John Wiley and Sons, New York, 1985). The *gpt* gene, on the other hand, may be used for both positive and negative selection in wild-type cells. Negative selection against the *gpt* gene in wild-type cells is possible using 6-thioxanthine, which is efficiently converted to a cytotoxic nucleotide analog by the bacterial *gpt* enzyme, but not by the cellular HPRT enzyme (Besnard et al., *Mol. Cell. Biol.* 7:4139, 1987).

More recently, attention has turned to selectable genes that may be incorporated into gene transfer vectors designed for use in human gene therapy. Gene therapy is a method for permanently curing a hereditary genetic disease which results from a defect in or absence of one or more genes. Collectively, such diseases result in significant morbidity and mortality. Examples of such genetic diseases include hemophilias A and B (caused by a deficiency of blood coagulation factors VIII and IX, respectively), alpha-1-antitrypsin deficiency, and adenosine deaminase deficiency. In each of these particular cases, the missing gene has been identified and its complementary DNA (cDNA) molecularly cloned (Wood et al., *Nature 312*:330, 1984; Anson et al., *Nature 315:683,* 1984; and Long et al., *Biochemistry* 23:4828, 1984; Daddona et al., J. *Biol. Chem.* 259:12101, 1984). While palliative therapy is available for some of these genetic diseases, often in the form of administration of blood products or blood transfusions, one way of permanently curing such genetic diseases is to introduce a replacement for the defective or missing gene back into the somatic cells of the patient, a process referred to as "gene therapy" (Anderson, *Science 226:401,* 1984). Gene therapy can also be used as a means for augmenting normal gene function, for example, by introducing a heterologous gene capable of modifying cellular activities or cellular phenotype, or alternatively, expressing a drug needed to treat a disease.

The process of gene therapy typically involves the steps of (1) removing somatic (non-germ) cells from the patient, (2) introducing into the cells *ex vivo* a replacement gene via an appropriate vector capable of expressing the replacement gene, and (3) transplanting or transfusing these cells back into the patient, where the replacement gene is expressed to provide some therapeutic benefit. Gene transfer into somatic cells for human gene therapy is presently achieved *ex vivo* (Kasid et al., *Proc. Natl Acad. Sci. USA* 87:473, 1990; Rosenberg et al., *N. Engl. J. Med.* 323:570, 1990), and this relatively inefficient process would be facilitated by the use of a dominant positive selectable gene for identifying and isolating those cells into which the replacement gene has been introduced before they are returned to the patient. The *neo* gene, for example, has been used to identify genetically modified cells used in human gene therapy.

In some instances, however, it is possible that the introduction of genetically modified cells may actually compromise the health of the patient. The ability to selectively eliminate genetically modified cells *in vivo* would provide an additional margin of safety for patients undergoing gene therapy, by permitting reversal of the procedure. This might be accomplished by incorporating into the vector a negative selectable (or 'suicide') gene that is capable of functioning in wild-type cells. Incorporation of a gene capable of conferring both dominant positive and negative selectable phenotpyes would ensure co-expression and co-regulation of the positive and negative selectable phenotypes, and would minimize the size of the vector. However, positive selection for the *gpt* gene in some instances requires precise selection conditions which may be difficult to determine. Moreover, the feasibility of using the gpt gene for *in vivo* negative selection has not yet been clearly established. For these reasons, co-expression of a dominant positive selectable phenotype and a negative selectable phenotype is typically achieved by co-expressing two different genes which separately encode other dominant positive and negative selectable functions, rather than using the *gpt* gene.

The existing strategies for co-expressing dominant positive and negative selectable phenotypes encoded by different genes often present complex challenges. As indicated above, the most widely used technique is to co-transfect two plasmids separately encoding two phenotypes (Wigler et al., *Cell 16*:777, 1979). However, the efficiency of co-transfer is rarely 100%, and the two genes may be subject to independent genetic or epigenetic regulation. A second strategy is to link the two genes on a single plasmid, or to place two independent transcription units into a viral vector. This method also suffers from the disadvantage that the genes may be independently regulated. In retroviral vectors, suppression of one or the other independent transcription unit may occur (Emerman and Temin, *Mol. Cell. Biol.* 6:792, 1986). In addition, in some circumstances there may be insufficient space to accommodate two functional transcription units within a viral vector, although retroviral vectors with functional multiple promoters have been successfully made (Overell et al., *Mol. Cell. Biol.* 8:1803, 1988). A third strategy is to express the two genes as a bicistronic mRNA using a single promoter. With this method, however, the distal open reading frame is often translated with variable (and usually reduced) efficiency (Kaufman et al., *EMBO J.* 6:187, 1987), and it is unclear how effective such an expression strategy would be in primary cells.

The present invention provides a method for more efficiently and reliably co-expressing a dominant positive selectable phenotype and a negative selectable phenotype encoded by different genes.

### SUMMARY OF THE INVENTION

The present invention provides a selectable fusion gene comprising a dominant positive selectable gene fused to and in reading frame with a negative selectable gene. The selectable fusion gene encodes a single bifunctional fusion protein which is capable of conferring a dominant positive selectable phenotype and a negative selectable phenotype on a cellular host. In a preferred embodiment, the selectable fusion gene comprises nucleotide sequences from the *hph* gene fused to nucleotide sequences from the HSV-I TK gene, referred to herein as the HyTK selectable fusion gene (Sequence Listing No. 1). The HyTK selectable fusion gene confers both hygromycin B resistance (Hm^{r}) for dominant positive selection and ganciclovir sensitivity (GCV^{s}) for negative selection.

The present invention also provides recombinant expression vectors, for example, retroviruses, which include the selectable fusion genes, and cells transduced with the recombinant expression vectors.

The selectable fusion genes of the present invention are expressed and regulated as a single genetic entity, permitting co-regulation and co-expression with a high degree of efficiency.

### BRIEF DESCRIPTION OF THE DRAWINGS

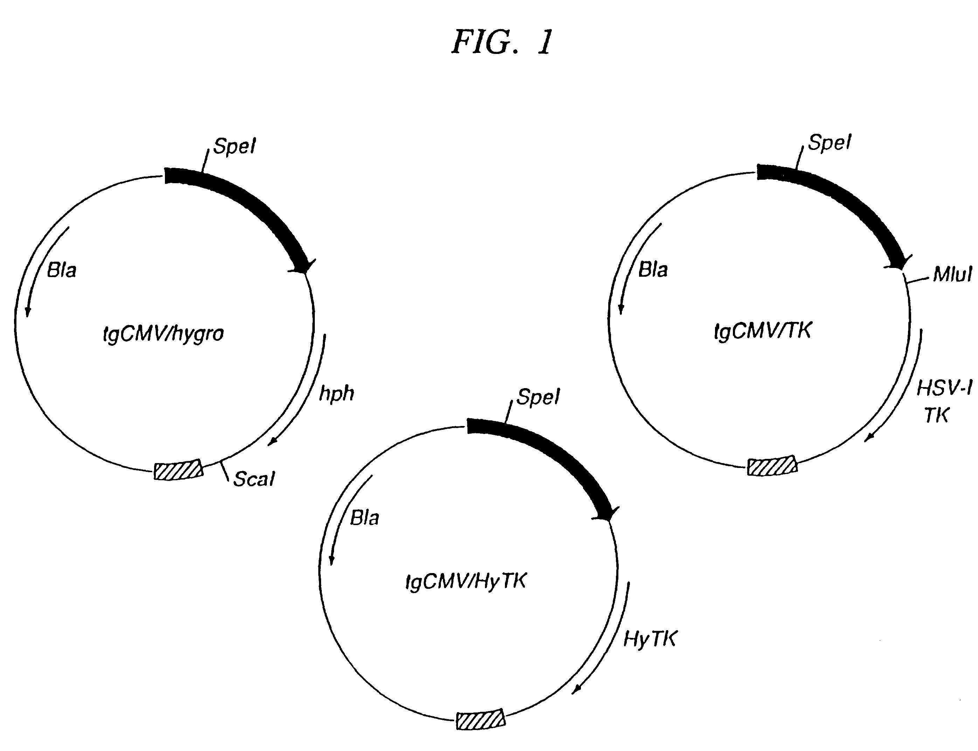

Figure 1 shows diagrams of the plasmids tgCMV/hygro, tgCMV/TK and tgCMV/HyTK which contain proviral structures used in the present invention. The three plasmids are identical, except for the genes inserted between the HCMV promoter (filled box) and the SV40 early region polyadenylation signal (hatched box).

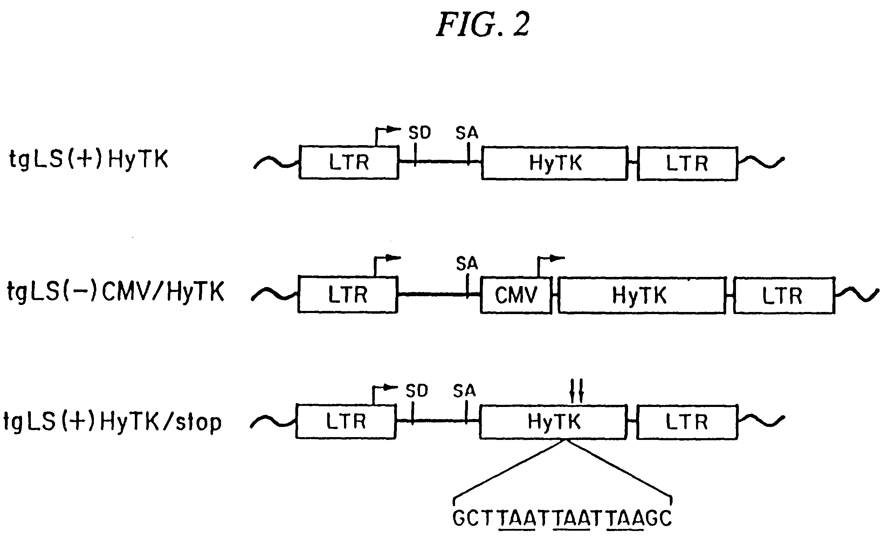

Figure 2 shows diagrams of the proviral structures from the plasmids shown in Figure 1. The horizontal arrows indicate transcriptional start sites and direction of transcription. The open box labeled LTR is the retroviral long terminal repeat. The viral splice donor is labeled SD and the acceptor sequences are labeled SA. The open box labeled CMV is the cytomegalovirus promoter. In tgLS(+)HyTK/stop, the positions of the two internal initiation codons retained in the HyTK selectable fusion gene are indicated by vertical arrows. The location at which the universal translation terminator oligonucleotide was inserted is also marked.

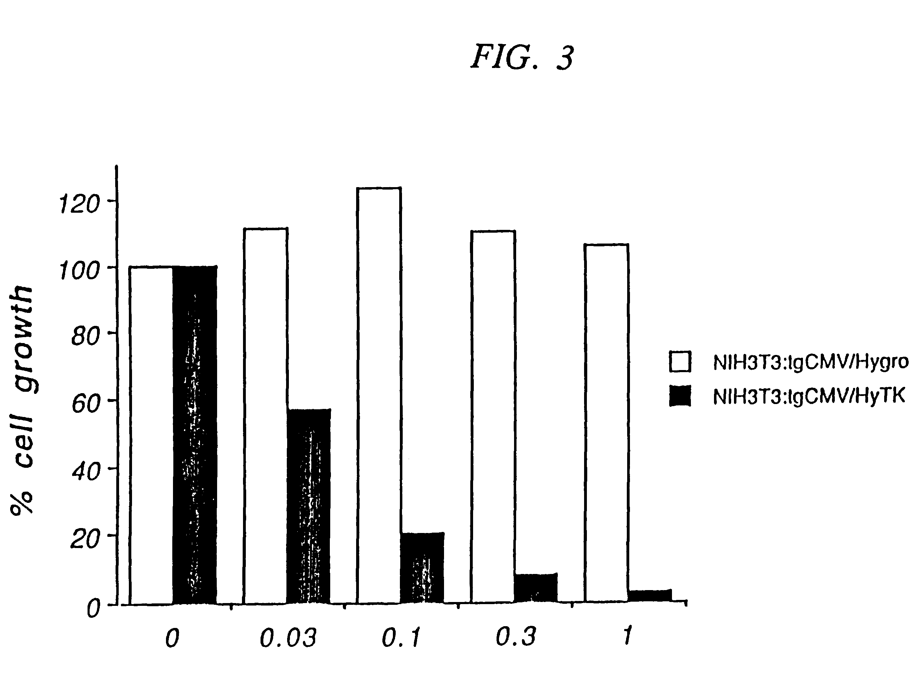

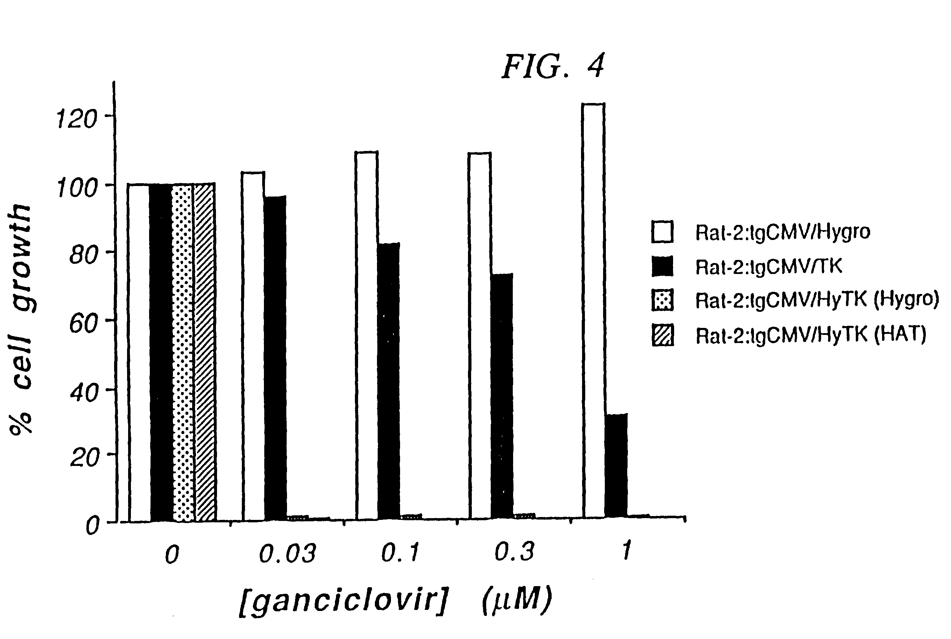

Figures 3 and 4 are graphs showing the results of a short-term proliferation assay in which the hygromycin resistant (Hm^{r}) NIH/3T3 cell pools and Hm^{r} and HAT resistant (HAT^{r}) Rat-2 cell pools were tested for ganciclovir sensitivity (GCV^{s}). Figure 3 shows that GCV inhibits growth of NIH/3T3 cells transfected with tgCMV/HyTK, but does not inhibit growth of NIH/3T3 cells transfected with tgCMV/Hygro. Figure 4 shows that GCV inhibits growth of Rat-2 cells transfected with tgCMV/HyTK (initially selected for Hm^{r} or HAT^{r}) even at the lowest concentrations of GCV, and also inhibits growth of Rat-2 cells transfected with tgCMV/TK, although at slightly higher concentrations. GCV did not inhibit growth of Rat-2 cells transfected with tgCMV/HYgro.

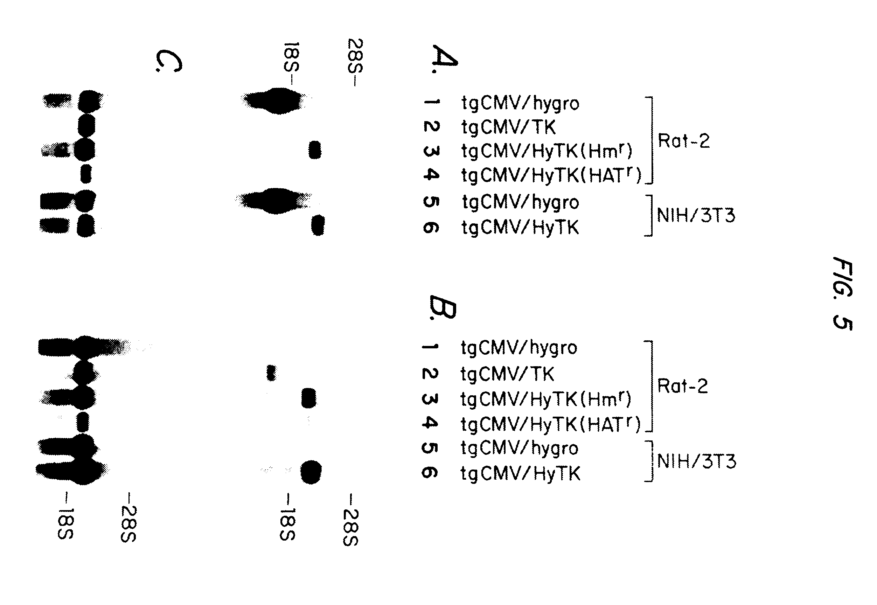

Figure 5 shows the results of Northern analysis of Hm^{r} and HAT^{r} cell pools. Polyadenylated mRNA was extracted from each Hm^{r} and HAT^{r} cell pool, and used to prepare Northern blots which were probed with sequences from the *hph* gene (Panel A), the HSV-I TK gene (Panel B), or the β-actin gene (Panel C) (for mRNA equivalence). The positions of the 28S and 18S ribosomal RNAs are indicated. The mRNA present in each lane was extracted from the following cells: Lane 1, Rat-2 cells transfected with tgCMV/hygro; Lane 2, Rat-2 cells transfected with tgCMV/TK; Lane 3, Rat-2 cells transfected with tgCMV/HyTK and selected for Hm^{r}; Lane 4, Rat-2 cells transfected with tgCMV/HyTK and selected for HAT^{r}; Lane 5, NIH/3T3 cells transfected with tgCMV/hygro; Lane 6, NIH/3T3 cells transfected with tgCMV/HyTK.

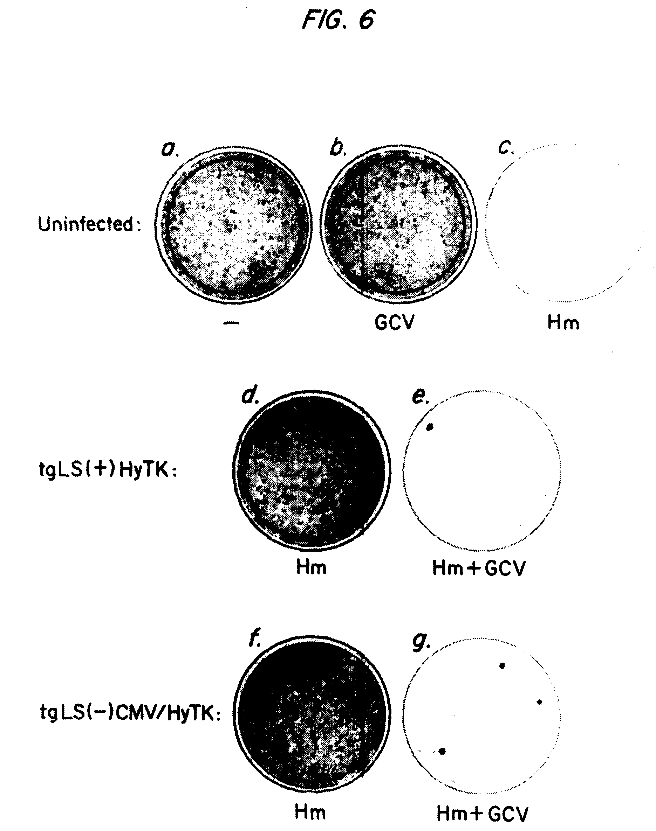

Figure 6 shows photographs of stained colonies of uninfected NIH/3T3 cells (plates a, b and c) and NIH/3T3 cells infected with the tgLS(+)HyTK (plates d and e) or tgLS(-) CMV/HyTK (plates f and g) retroviruses. The cells were grown in medium alone or medium supplemented with GCV, Hm or Hm plus GCV in a long-term proliferation assay. The data show that uninfected NIH/3T3 cells were resistant to GCV and grew to confluence (plate b), but were killed by Hm (plate c). Growth of NIH/3T3 cells infected with the tgLS(+)HyTK and tgLS(-) CMV/HyTK retroviruses and grown in the presence of Hm (plates d and f) was inhibited by GCV (plates e and g).

### DETAILED DESCRIPTION OF THE INVENTION

SEQ ID NO: 1 and SEQ ID NO:2 (appearing immediately prior to the claims) show specific embodiments of the nucleotide sequence and corresponding amino acid sequence of the HyTK selectable fusion gene of the present invention. The HyTK selectable fusion gene shown in the Sequence Listing comprises sequences from the *hph* gene (nucleotides 1-971) linked to sequences from the HSV-I TK gene (nucleotides 972-2076).

### Definitions

As used herein, the term "selectable fusion gene" refers to a nucleotide sequence comprising a dominant positive selectable gene which is fused to and in reading frame with a negative selectable gene and which encodes a single bifunctional fusion protein which is capable of conferring a dominant positive selectable phenotype and a negative selectable phenotype on a cellular host. A "dominant positive selectable gene" refers to a sequence of nucleotides which encodes a protein conferring a dominant positive selectable phenotype on a cellular host, and is discussed and exemplified in further detail below. A "negative selectable gene" refers to a sequence of nucleotides which encodes a protein conferring a negative selectable phenotype on a cellular host, and is also discussed and exemplified in further detail below. A "selectable gene" refers generically to dominant positive selectable genes and negative selectable genes.

A selectable gene is "fused to and in reading frame with" another selectable gene if the expression products of the selectable genes (i.e., the proteins encoded by the selectable genes) are fused by a peptide bond and at least part of the biological activity of each of the two proteins is retained. With reference to the HyTK selectable fusion gene disclosed herein, for example, the *hph* gene (encoding hygromycin-B phosphotransferase, which confers the dominant positive selectable phenotype of hygromycin resistance (Hm^{r})) is fused to and in reading frame with the HSV-I TK gene (encoding Herpes Simplex Virus Type I thymidine kinase, which confers a negative selectable phenotype of ganciclovir sensitivity, or (GCV^{s})) if the *hph* and HSV-I TK proteins are fused by a peptide bond and expressed as a single bifunctional fusion protein. The component selectable gene sequences of the present invention are preferably contiguous; however, it is possible to construct selectable fusion genes in which the component selectable gene sequences are separated by internal nontranslated nucleotide sequences, such as introns. For purposes of the present invention, such noncontiguous selectable gene sequences are considered to be fused, provided that expression of the selectable fusion gene results in a single bifunctional fusion protein in which the expression products of the component selectable gene sequences are fused by a peptide bond.

"Nucleotide sequence" refers to a heteropolymer of deoxyribonucleotides or ribonucleotides, such as a DNA or RNA sequence. Nucleotide sequences may be in the form of a separate fragment or as a component of a larger construct. Preferably, the nucleotide sequences are in a quantity or concentration enabling identification, manipulation, and recovery of the sequence by standard biochemical methods, for example, using a cloning vector. Recombinant nucleotide sequences are the product of various combinations of cloning, restriction, and ligation steps resulting in a construct having a structural coding sequence distinguishable from homologous sequences found in natural systems. Generally, nucleotide sequences encoding the structural coding sequence, for example, the selectable fusion genes of the present invention, can be assembled from nucleotide fragments and short oligonucleotide linkers, or from a series of oligonucleotides, to provide a synthetic gene which is capable of being expressed in a recombinant transcriptional unit. Such sequences are preferably provided in the form of an open reading frame uninterrupted by internal nontranslated sequences, or introns, which are typically present in eukaryotic genes. Genomic DNA containing the relevant selectable gene sequences is preferably used to obtain appropriate nucleotide sequences encoding selectable genes; however, cDNA fragments may also be used. Sequences of non-translated DNA may be present 5' or 3' from the open reading frame or within the open reading frame, provided such sequences do not interfere with manipulation or expression of the coding regions. Some genes, however, may include introns which are necessary for proper expression in certain hosts, for example, the HPRT selectable gene includes introns which are necessary for expression in embryonal stem (ES) cells. As suggested above, the nucleotide sequences of the present invention may also comprise RNA sequences, for example, where the nucleotide sequences are packaged as RNA in a retrovirus for infecting a cellular host. The use of retroviral expression vectors is discussed in greater detail below.

The term "recombinant expression vector" refers to a replicable unit of DNA or RNA in a form which is capable of being transduced into a target cell by transfection or viral infection, and which codes for the expression of a selectable fusion gene which is transcribed into mRNA and translated into protein under the control of a genetic element or elements having a regulatory role in gene expression, such as transcription and translation initiation and termination sequences. The recombinant expression vectors of the present invention can take the form of DNA constructs replicated in bacterial cells and transfected into target cells directly, for example, by calcium phosphate precipitation, electroporation or other physical transfer methods. The recombinant expression vectors which take the form of RNA constructs may, for example, be in the form of infectious retroviruses packaged by suitable "packaging" cell lines which have previously been transfected with a proviral DNA vector and produce a retrovirus containing an RNA transcript of the proviral DNA. A host cell is infected with the retrovirus, and the retroviral RNA is replicated by reverse transcription into a double-stranded DNA intermediate which is stably integrated into chromosomal DNA of the host cell to form a provirus. The provirus DNA is then expressed in the host cell to produce polypeptides encoded by the DNA. The recombinant expression vectors of the present invention thus include not only RNA constructs present in the infectious retrovirus, but also copies of proviral DNA, which include DNA reverse transcripts of a retrovirus RNA genome stably integrated into chromosomal DNA in a suitable host cell, or cloned copies thereof, or cloned copies of unintegrated intermediate forms of retroviral DNA. Proviral DNA includes transcriptional elements in independent operative association with selected structural DNA sequences which are transcribed into mRNA and translated into protein when proviral sequences are expressed in infected host cells. Recombinant expression vectors used for direct transfection will include DNA sequences enabling replication of the vector in bacterial host cells. Various recombinant expression vectors suitable for use in the present invention are described below.

"Transduce" means introduction of a recombinant expression vector containing a selectable fusion gene into a cell. Transduction methods may be physical in nature (i.e., transfection), or they may rely on the use of recombinant viral vectors, such as retroviruses, encoding DNA which can be transcribed to RNA, packaged into infectious viral particles and used to infect target cells and thereby deliver the desired genetic material (i.e., infection). Many different types of mammalian gene transfer and recombinant expression vectors have been developed (see, e.g., Miller and Calos, eds., "Gene Transfer Vectors for Mammalian Cells," *Current Comm. Mol. Biol.,* (Cold Spring Harbor Laboratory, New York, 1987)). Naked DNA can be physically introduced into mammalian cells by transfection using any one of a number of techniques including, but not limited to, calcium phosphate transfection (Berman et al., *Proc. Natl. Acad. Sci. USA 84* 81: 7176, 1984), DEAE-Dextran transfection (McCutchan et al., J. *Natl. Cancer Inst. 41*:351, 1986; Luthman et al., *Nucl. Acids Res.* 11:1295, 1983), protoplast fusion (Deans et al., *Proc. Natl. Acad. Sci. USA 84* 81: 1292, 1984), electroporation (Potter et al., *Proc. Natl. Acad. Sci. USA 84* 81: 7161, 1984), lipofection (Felgner et al., *Proc. Natl. Acad. Sci. USA* 84:7413, 1987), polybrene transfection (Kawai and Nishzawa, *Mol. Cell. Biol* 4:1172, 1984) and direct gene transfer by laser micropuncture of cell membranes (Tao et al., *Proc. Natl. Acad. Sci. USA 84* 84:4180, 1987). Various infection techniques have been developed which utilize recombinant infectious virus particles for gene delivery. This represents a preferred approach to the present invention. The viral vectors which have been used in this way include virus vectors derived from simian virus 40 (SV40; Karlsson et al., *Proc. Natl. Acad. Sci. USA* 84 82:158, 1985), adenoviruses (Karlsson et al., *EMBO* J. 5:2377, 1986), adeno-associated virus (LaFace et al., *Virology 162*:483, 1988) and retroviruses (Coffin, 1985, p17-71 in Weiss et al (eds.), *RNA Tumor Viruses,* 2nd ed., Vol 2, Cold Spring Harbor Laboratory, New York). Thus, gene transfer and expression methods are numerous but essentially function to introduce and express genetic material in mammalian cells. Several of the above techniques have been used to transduce hematopoietic or lymphoid cells, including calcium phosphate transfection (Berman et al., *supra,* 1984), protoplast fusion (Deans et al., *supra,* 1984), electroporation (Cann et al., *Oncogene* 3:123, 1988), and infection with recombinant adenovirus (Karlsson et al., *supra;* Ruether et al., *Mol. Cell. Biol.* 6:123, 1986) adeno-associated virus (LaFace et al., *supra)* and retrovirus vectors (Overell et al., *Oncogene* 4:1425, 1989). Primary T lymphocytes have been successfully transduced by electroporation (Cann et al., *supra,* 1988) and by retroviral infection (Nishihara et al., *Cancer Res.* 48:4730, 1988; Kasid et al., *supra,* 1990).

### Construction of Selectable Fusion Genes

The selectable fusion genes of the present invention comprise a dominant positive selectable gene fused to a negative selectable gene. A selectable gene will generally comprise, for example, a gene encoding a protein capable of conferring an antibiotic resistance phenotype or supplying an autotrophic requirement (for dominant positive selection), or activating a toxic metabolite (for negative selection). A DNA sequence encoding a bifunctional fusion protein is constructed using recombinant DNA techniques to assemble separate DNA fragments encoding a dominant positive selectable gene and a negative selectable gene into an appropriate expression vector. The 3' end of one selectable gene is ligated to the 5' end of the other selectable gene, with the reading frames of the sequences in frame to permit translation of the mRNA sequences into a single biologically active bifunctional fusion protein. The selectable fusion gene is expressed under control of a single promoter.

The dominant positive selectable gene is any gene which, upon being transduced into a host cell, expresses a dominant phenotype permitting positive selection of stable transductants. Selection of stable transductants can be carried out, for example, using the hygromycin-B phosphotransferase gene (*hph*) which confers the selectable phenotype of hygromycin resistance (Hm^{r}) (Santerre et al., *Gene 30:147,* 1984; Sugden et al., *Mol. Cell. Biol.* 5:410, 1985; obtainable from plasmid pHEBol, under ATCC Accession No. 39820). Hygromycin B is an aminoglycoside antibiotic that inhibits protein synthesis by disrupting translocation and promoting mistranslation. The *hph* gene confers Hm^{r} to cells transduced with the *hph* gene by phosphorylating and detoxifying the antibiotic hygromycin B. Other acceptable dominant positive selectable genes include the following: the aminoglycoside phosphotransferase gene *(neo* or *aph)* from Tn5 which codes for resistance to the antibiotic G418 (Colbere-Garapin et al., J. *Mol. Biol. 150:1,* 198; Southern and Berg, J. *Mol. Appl. Genet.* 1:327, 1982); the xanthine-guanine phosphoribosyl transferase gene *(gpt)* from *E. coli* encoding resistance to mycophenolic acid (Mulligan and Berg, *Proc. Natl. Acad. Sci USA* 78:2072, 1981); the dihydrofolate reductase (DHFR) gene from murine cells or *E. coli* which is necessary for biosynthesis of purines and can be competitively inhibited by the drug methotrexate (MTX) to select for cells constitutively expressing increased levels of DHFR (Simonsen and Levinson, *Proc. Natl. Acad. Sci.* (*U.S.A.*) 80:2495, 1983; Simonsen et al., *Nucl. Acids. Res.* 16:2235, 1988); the S. *typhimurium* histidinol dehydrogenase *(hisD)* gene (Hartman, et al, *Proc. Natl. Acad. Sci. (USA)* 85:8047, 1988); the *E. coli* tryptophan synthase β subunit *(trpB)* gene (Hartman et al., *supra);* the puromycin-N-acetyl transferase (*pac*) gene (Vara et al., *Nucl. Acids Res. 14*:4117, 1986); the adenosine deaminase (ADA) gene (Daddona et al., J. *Biol. Chem. 259*:12101, 1984); the multi-drug resistance (MDR) gene (Kane et al., *Gene 84*:439, 1989); the mouse ornithine decarboxylase (OCD) gene (Gupba and Coffino, J. *Biol. Chem.* 260:2941, 1985); the *E. coli* aspartate transcarbamylase catalytic subunit (*pyrB*) gene (Ruiz and Wahl, *Mol. Cell. Biol.* 6:3050, 1986); and the *E. coli asnA* gene, encoding asparagine sythetase (Cartier et al., *Mol. Cell. Biol.* 7:1623, 1987).

The negative selectable gene is any gene which, upon being transduced into a host cell, expresses a phenotype permitting negative selection (i.e., elimination) of stable transductants. In preferred embodiments, the negative selectable gene used in the fusion genes of the present invention is the *Herpes simplex* virus type I thymidine kinase (HSV-I TK) gene (Wigler et al., *Cell 11*:223, 1977; McKnight et al., *Nucl. Acids Res.* 8:5931, 1980; Preston et al., J. *Virol. 38:593,* 1981; Wagner et al., *Proc. Natl. Acad. Sci. USA 78:1441,* 1981) which confers ganciclovir sensitivity (GCV^{s}) (St. Clair et al., *Antimicrob. Agents Chemother. 31:844,* 1987). The HSV-I TK gene is available from Bethesda Research Labs (Catalog No. BRL 5365 SA). Negative selection can also be performed, for example, using the cellular hypoxanthine phosphoribosyltransferase (HPRT) gene (Jolly et al., *Proc. Natl. Acad. Sci. USA* 80:477, 1983; Fenwick, "The HGPRT System", pp. 333-373, M. Gottesman (ed.), *Molecular Cell Genetics,* (John Wiley and Sons, New York, 1985)) and the cellular adenine phosphoribosyltransferase (APRT) gene (Wigler et al., *Proc. Natl. Acad. Sci. USA* 76:1373, 1979; Taylor et al., "The APRT System", pp., 311-332, M. Gottesman (ed.), *Molecular Cell Genetics,* (John Wiley and Sons, New York, 1985)); and the *E. coli gpt* gene (Besnard et al., *Mol. Cell. Biol.* 7:4139, 1987).

Due to the degeneracy of the genetic code, there can be considerable variation in nucleotide sequences encoding the same amino acid sequence; exemplary DNA embodiments are those corresponding to the nucleotide sequences shown in Sequence Listing No. 1. Such variants will have modified DNA or amino acid sequences, having one or more substitutions, deletions, or additions, the net effect of which is to retain biological activity, and may be substituted for the specific sequences disclosed herein. The sequences of selectable fusion genes comprising *hph* and TK are equivalent if they contain all or part of the sequences of *hph* and HSV-I TK and are capable of hybridizing to the nucleotide sequence of Sequence Listing No. 1 under moderately stringent conditions (50°C, 2 X SSC) and express a biologically active fusion protein. A "biologically active" fusion protein will share sufficient amino acid sequence similarity with the specific embodiments of the present invention disclosed herein to be capable of conferring the selectable phenotypes of the component selectable genes.

In a preferred embodiment, sequences from the bacterial hygromycin phosphotransferase (*hph*) gene are fused with sequences from the HSV-I TK gene. The resulting selectable fusion gene (referred to as the HyTK selectable fusion gene) encodes a bifunctional fusion protein that confers Hm^{r} and GCV^{s}, and provides a means by which dominant positive and negative selectable phenotypes may be expressed and regulated as a single genetic entity. The HyTK selectable fusion gene is therefore a useful addition to the existing panel of selectable genes available for use in animal cells, because it allows both dominant positive and negative selection in wild-type cells.

### Recombinant Expression Vectors

The selectable fusion genes of the present invention are utilized to identify, isolate or eliminate host cells into which the selectable fusion genes are introduced. The selectable fusion genes are introduced into the host cell by transducing into the host cell a recombinant expression vector which contains the selectable fusion gene. Such host cells include cell types from higher eukaryotic origin, such as mammalian or insect cells, or cell types from lower prokaryotic origin, such as bacterial cells, for example, *E. coli.*

As indicated above, such selectable fusion genes are preferably introduced into a particular cell as a component of a recombinant expression vector which is capable of expressing the selectable fusion gene within the cell and conferring a selectable phenotype. Such recombinant expression vectors generally include synthetic or natural nucleotide sequences comprising the selectable fusion gene operably linked to suitable transcriptional or translational control sequences, for example, an origin of replication, optional operator sequences to control transcription, a suitable promoter and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences. Such regulatory sequences can be derived from mammalian, viral, microbial or insect genes. Nucleotide sequences are operably linked when they are functionally related to each other. For example, a promoter is operably linked to a selectable fusion gene if it controls the transcription of the selectable fusion gene; or a ribosome binding site is operably linked to a selectable fusion gene if it is positioned so as to permit translation of the selectable fusion gene into a single bifunctional fusion protein. Generally, operably linked means contiguous.

Specific recombinant expression vectors for use with mammalian, bacterial, and yeast cellular hosts are described by Pouwels et al. *(Cloning Vectors: A Laboratory Manual,* Elsevier, New York, 1985) and are well-known in the art. A detailed description of recombinant expression vectors for use in animal cells can be found in Rigby, *J. Gen. Virol.* 64:255, 1983; Elder et al., *Ann. Rev. Genet.* 15:295, 1981; and Subramani et al., *Anal. Biochem. 135:1,* 1983. Appropriate recombinant expression vectors may also include viral vectors, in particular retroviruses (discussed in detail below).

The selectable fusion genes of the present invention are preferably placed under the transcriptional control of a strong enhancer and promoter expression cassette. Examples of such expression cassettes include the human cytomegalovirus immediate-early (HCMV-IE) promoter (Boshart et al., *Cell 41*:521, 1985), the β-actin promoter (Gunning et al., *Proc. Natl. Acad. Sci. (USA) 84*:5831, 1987), the histone H4 promoter (Guild et al., *J. Virol. 62*:3795, 1988), the mouse metallothionein promoter (McIvor et al., *Mol. Cell. Biol. 7*:838, 1987), the rat growth hormone promoter (Miller et al., *Mol. Cell. Biol.* 5:431, 1985), the human adenosine deaminase promoter (Hantzapoulos et al., *Proc. Natl. Acad. Sci. USA 86*:3519, 1989) the HSV *tk* promoter (Tabin et al., *Mol. Cell. Biol. 2*:426, 1982), the α-1 antitrypsin enhancer (Peng et al., *Proc. Natl. Acad. Sci. USA 85*:8146, 1988) and the immunoglobulin enhancer/promoter (Blankenstein et al., *Nucleic Acid Res. 16*:10939, 1988), the SV40 early or late promoters, the Adenovirus 2 major late promoter, or other viral promoters derived from polyoma virus, bovine papilloma virus, or other retroviruses or adenoviruses. The promoter and enhancer elements of immunoglobulin (Ig) genes confer marked specificity to B lymphocytes (Banerji et al., *Cell 33*:729, 1983; Gillies et al., *Cell* 33:717, 1983; Mason et al., *Cell 41:479,* 1985), while the elements controlling transcription of the β-globin gene function only in erythroid cells (van Assendelft et al., *Cell* 56:969, 1989). Using well-known restriction and ligation techniques, appropriate transcriptional control sequences can be excised from various DNA sources and integrated in operative relationship with the intact selectable fusion genes to be expressed in accordance with the present invention. Thus, many transcriptional control sequences may be used successfully in retroviral vectors to direct the expression of inserted genes in infected cells.

### Retroviruses

Retroviruses can be used for highly efficient transduction of the selectable fusion genes of the present invention into eukaryotic cells and are preferred for the delivery of a selectable fusion gene into primary cells. Moreover, retroviral integration takes place in a controlled fashion and results in the stable integration of one or a few copies of the new genetic information per cell.

Retroviruses are a class of viruses whose genome is in the form of RNA. The genomic RNA of a retrovirus contains *trans*-acting gene sequences coding for three viral proteins: a structural protein *gag* which associates with the RNA in the core of the virus particle; the reverse transcriptase *pol* which makes the DNA complement; and a envelope glycoprotein *env* which resides in the lipoprotein envelope of the particles and binds the virus to the surface of host cells on infection. Replication of the retrovirus is regulated by *cis-acting* elements, such as the promoter for transcription of the proviral DNA and other nucleotide sequences necessary for viral replication. The *cis-acting* elements are present in or adjacent to two identical untranslated long terminal repeats (LTRs) of about 600 base pairs present at the 5' and 3' ends of the retroviral genome. Retroviruses replicate by copying their RNA genome by reverse transcription into a double-stranded DNA intermediate, using a virus-encoded, RNA-directed DNA polymerase, or reverse transcriptase. The DNA intermediate is integrated into chromosomal DNA of an avian or mammalian host cell. The integrated retroviral DNA is called a provirus. The provirus serves as template for the synthesis of RNA chains for the formation of infectious virus particles. Forward transcription of the provirus and assembly into infectious virus particles occurs in the presence of an appropriate helper virus having endogenous *trans*-acting genes required for viral replication.

Retroviruses are used as vectors by replacing one or more of the endogenous trans-acting genes of a proviral form of the retrovirus with a recombinant therapeutic gene or, in the case of the present invention, a selectable fusion gene, and then transducing the recombinant provirus into a cell. The *trans*-acting genes include the *gag*, *pol* and *env* genes which encode, respectively, proteins of the viral core, the enzyme reverse transcriptase and constituents of the envelope protein, all of which are necessary for production of intact virions. Recombinant retroviruses deficient in the trans-acting *gag*, *pol* or *env* genes cannot synthesize essential proteins for replication and are accordingly replication-defective. Such replication-defective recombinant retroviruses are propagated using packaging cell lines. These packaging cell lines contain integrated retroviral genomes which provide all *trans*-acting gene sequences necessary for production of intact virions. Proviral DNA sequences which are transduced into such packing cells lines are transcribed into RNA and encapsidated into infectious virions containing the selectable fusion gene (and/or therapeutic gene), but, lacking the *trans*-acting gene products *gag, pol* and *env,* cannot synthesize the necessary *gag*, *pol* and *env* proteins for encapsidating the RNA into particles for infecting other cells. The resulting infectious retrovirus vectors can therefore infect other cells and integrate a selectable fusion gene into the cellular DNA of a host cell, but cannot replicate. Mann et al. *(Cell 33*:153, 1983), for example, describe the development of various packaging cell lines (e.g., ψ2) which can be used to produce helper virus-free stocks of recombinant retrovirus. Encapsidation in a cell line harboring *trans*-acting elements encoding an ecotropic viral envelope (e.g., ψ2) provides ecotropic (limited host range) progeny virus. Alternatively, assembly in a cell line containing amphotropic packaging genes (e.g., PA317, ATCC CRL 9078; Miller and Buttimore, *Mol. Cell. Biol. 6*:2895, 1986) provides amphotropic (broad host range) progeny virus.

Numerous provirus constructs have been used successfully to express foreign genes (see, e.g., Coffin, in Weiss et al. (eds.), *RNA Tumor Viruses,* 2nd Ed., Vol. 2, (Cold Spring Harbor Laboratory, New York, 1985, pp. 17-71). Most proviral elements are derived from murine retroviruses. Retroviruses adaptable for use in accordance with the present invention can, however, be derived from any avian or mammalian cell source. Suitable retroviruses must be capable of infecting cells which are to be the recipients of the new genetic material to be transduced using the retroviral vector. Examples of suitable retroviruses include avian retroviruses, such as avian erythroblastosis virus (AEV), avian leukosis virus (ALV), avian myeloblastosis virus (AMV), avian sarcoma virus (ASV), Fujinami sarcoma virus (FuSV), spleen necrosis virus (SNV), and Rous sarcoma virus (RSV); bovine leukemia virus (BLV); feline retroviruses, such as feline leukemia virus (FeLV) or feline sarcoma virus (FeSV); murine retroviruses, such as murine leukemia virus (MuLV); mouse mammary tumor virus (MMTV), and murine sarcoma virus (MSV); and primate retroviruses, such as human T-cell lymphotropic viruses 1 and 2 (HTLV-1, and -2), and simian sarcoma virus (SSV). Many other suitable retroviruses are known to those skilled in the art. A taxonomy of retroviruses is provided by Teich, in Weiss et al. (eds.), *RNA Tumor Viruses,* 2d ed., Vol. 2 (Cold Spring Harbor Laboratory, New York, 1985, pp. 1-160). Preferred retroviruses for use in connection with the present invention are the murine retroviruses known as Moloney murine leukemia virus (MoMLV), Moloney murine sarcoma virus (MoMSV), Harvey murine sarcoma virus (HaMSV) and Kirsten murine sarcoma virus (KiSV). the sequences required to construct a retroviral vector from the MoMSV genome can be obtained in conjunction with a pBR322 plasmid sequence such as pMV (ATCC 37190), while a cell line producer of KiSV in K-BALB cells has been deposited as ATCC CCL 163.3. A deposit of pRSVneo, derived from pBR322 including the RSV LTR and an intact neomycin drug resistance marker is available from ATCC under Accession No. 37198. Plasmid pPB101 comprising the SNV genome is available as ATCC 45012. The viral genomes of the above retroviruses are used to construct replication-defective retrovirus vectors which are capable of integrating their viral genomes into the chromosomal DNA of an infected host cell but which, once integrated, are incapable of replication to provide infectious virus, unless the cell in which it is introduced contains other proviral elements encoding functional active *trans*-acting viral proteins.

The selectable fusion genes of the present invention which are transduced by retroviruses are expressed by placing the selectable fusion gene under the transcriptional control of the enhancer and promoter incorporated in the retroviral LTR, or by placing them under the control of a heterologous transcriptional control sequences inserted between the LTRs. Use of both heterologous transcriptional control sequences and the LTR transcriptional control sequences enables coexpression of a therapeutic gene and a selectable fusion gene in the vector, thus allowing selection of cells expressing specific vector sequences encoding the desired therapeutic gene product. Obtaining high-level expression may require placing the therapeutic gene and/or selectable fusion gene within the retrovirus under the transcriptional control of a strong heterologous enhancer and promoter expression cassette. Many different heterologous enhancers and promoters have been used to express genes in retroviral vectors. Such enhancers or promoters can be derived from viral or cellular sources, including mammalian genomes, and are preferably constitutive in nature. Such heterologous transcriptional control sequences are discussed above with reference to recombinant expression vectors. To be expressed in the transduced cell, DNA sequences introduced by any of the above gene transfer methods are usually expressed under the control of an RNA polymerase II promoter.

Particularly preferred recombinant expression vectors for use in retroviruses include pLXSN, pLNCX and pLNL6, and derivatives thereof, which are described by Miller and Rosman, *Biotechniques* 7:980, 1989. These vectors are capable of expressing heterologous DNA under the transcriptional control of the retroviral LTR or the CMV promoter, and the *neo* gene under the control of the SV40 early region promoter or the retroviral LTR. For use in the present invention, the *neo* gene is replaced with the bifunctional selectable fusion genes disclosed herein, such as the HyTK selectable fusion gene. Construction of useful replication-defective retroviruses is a matter of routine skill. The resulting recombinant retroviruses are capable of integration into the chromosomal DNA of an infected host cell, but once integrated, are incapable of replication to provide infectious virus, unless the cell in which it is introduced contains another proviral insert encoding functionally active *trans*-acting viral proteins.

### Uses of Bifunctional Selectable Fusion Genes

The selectable fusion genes of the present invention are particularly preferred for use in gene therapy as a means for identifying, isolating or eliminating cells, such as somatic cells, into which the selectable fusion genes are introduced. In gene therapy, somatic cells are removed from a patient, transduced with a recombinant expression vector containing a therapeutic gene and the selectable fusion gene of the present invention, and then reintroduced back into the patient. Somatic cells which can be used as vehicles for gene therapy include hematopoietic (bone marrow-derived) cells, keratinocytes, hepatocytes, endothelial cells and fibroblasts (Friedman, *Science 244*:1275, 1989). Alternatively, gene therapy can be accomplished through the use of injectable vectors which transduce somatic cells *in vivo.* The feasibility of gene transfer in humans has been demonstrated (Kasid et al., *Proc. Natl. Acad. Sci. USA 87:473,* 1990; Rosenberg et al., *N. Engl. J. Med. 323:570,* 1990).

The selectable fusion genes of the present invention are particularly useful for eliminating genetically modified cells *in vivo. In vivo* elimination of cells expressing a negative selectable phenotype is particularly useful in gene therapy as a means for ablating a cell graft, thereby providing a means for reversing the gene therapy procedure. For example, it has been shown that administration of the anti-herpes virus drug ganciclovir to transgenic animals expressing the HSV-I TK gene from an immunoglobulin promoter results in the selective ablation of cells expressing the HSV-I TK gene (Heyman et al., *Proc. Natl. Acad. Sci* (*USA*) *86*:2698, 1989). Using the same transgenic mice, GCV has also been shown to induce full regression of Abelson leukemia virus-induced lymphomas ((Moolten et al., *Human Gene Therapy* 1:125, 1990). In a third study, in which a murine sarcoma (K3T3) was infected with a retrovirus expressing HSV-I TK and transplanted into syngeneic mice, the tumors induced by the sarcoma cells were completely eradicated following treatment with GCV (Moolten and Wells, J. *Natl. Cancer Inst. 82*:297, 1990).

The bifunctional selectable fusion genes of the present invention can also be used to facilitate gene modification by homologous recombination. Reid et al., *Proc. Natl. Acad. Sci. USA 87*:4299, 1990 has recently described a two-step procedure for gene modification by homologous recombination in ES cells ("in-out" homologous recombination) using the HPRT gene. Briefly, this procedure involves two steps: an "in" step in which the HPRT gene is embedded in target gene sequences, transfected into HPRT- host cells and homologous recombinants having incorporated the HPRT gene into the target locus are identified by their growth in HAT medium and genomic analysis using PCR. In a second "out" step, a construct containing the desired replacement sequences embedded in the target gene sequences (but without the HPRT gene) is transfected into the cells and homologous recombinants having the replacement sequences (but not the HPRT gene) are isolated by negative selection against HPRT⁺ cells. Although this procedure allows the introduction of subtle mutations into a target gene without introducing selectable gene sequences into the target gene, it requires positive selection of transformants in a HPRT- cell line, since the HPRT gene is recessive for positive selection. Also, due to the inefficient expression of the HPRT gene in ES cells, it is necessary to use a large 9-kbp HPRT mini-gene which complicates the construction and propagation of homologous recombination vectors. The selectable fusion genes of the present invention provide an improved means whereby "in-out" homologous recombination may be performed. Because the selectable fusion genes of the present invention are dominant for positive selection, any wild-type cell may be used (i.e., one is not limited to use of cells deficient in the selectable phenotype). Moreover, the size of the vector containing the selectable fusion gene is reduced significantly relative to the large HPRT mini-gene.

By way of illustration, the HyTK selectable fusion gene is used as follows: In the first "in" step, the HyTK selectable fusion gene is embedded in target gene sequences, transfected into a host cell, and homologous recombinants having incorporated the HyTK selectable fusion gene into the target locus are identified by their growth in medium containing Hm followed by genome analysis using PCR. The HyTK⁺ cells are then used in the second "out" step, in which a construct containing the desired replacement sequences embedded in the target gene sequences (but without the HyTK selectable fusion gene) is transfected into the cells. Homologous recombinants are isolated by selective elimination of HyTK⁺ cells using ganciclovir followed by genome analysis using PCR.

### EXAMPLES

### Example 1

### Construction and Characterization of Plasmid Vectors Containing HyTK Selectable Fusion Gene

A. Construction of the Bifunctional HyTK Selectable Fusion Gene. The *hph* and HSV-I TK genes were first placed under the regulatory control of the HCMV promoter in tgCMV/hygro and tgCMV/TK, respectively. Plasmid tgCMV/hygro (Figure 1) consists of the following elements: the BalI-SstII fragment containing the HCMV IE94 promoter (Boshart et al., *Cell 41*:521, 1985); an oligonucleotide containing a sequence conforming to a consensus translation initiation sequence for mammalian cells (GCCGCCACC ATG) (Kozak, *Nucl. Acids Res.* 15:8125, 1987); nucleotides 234-1256 from the *hph* gene (Kaster et al., *Nucl. Acids Res. 11*:6895, 1983), encoding hygromycin phosphotransferase; the BclI-BamHI fragment from the SV40 genome (Tooze, J., ed., *Molecular Biology of Tumor Viruses, 2nd Ed. DNA Tumor Viruses.* Cold Spring Harbor Laboratory, New York, 1981), containing the SV40 early region polyadenylation sequence; the NruI-AlwNI fragment from pML2d (Lusky and Botchan, *Nature* 293:79, 1981), containing the bacterial replication origin; and the AlwNI-AatII fragment from pGEM1 (Promega Corporation), containing the β-lactamase gene.

Plasmid tgCMV/TK (Figure 1) is similar to tgCMV/hygro, but contains nucleotides 519-1646 from the HSV-I TK gene (Wagner et al., *Proc. Natl. Acad. Sci. USA 78:1441,* 1981) in place of the *hph* gene.

Plasmid tgCMV/HyTK (Figure 1), containing the selectable fusion gene comprising the *hph* gene and the HSV-I TK gene, was constructed by inserting the 1644-bp SpeI-ScaI fragment from tgCMV/hygro between the SpeI and MluI sites of tgCMV/TK. Before ligation, the MluI site in the HSV-I TK gene was treated with T4 DNA polymerase to allow blunt end ligation with the ScaI site, thus preserving the open reading frame. Translation of this fused gene (referred to as the HyTK selectable fusion gene) is expected to generate a single bifunctional fusion protein, consisting of amino acids 1-324 from the *hph* protein and amino acids 10-376 from the HSV-I TK protein. The C-terminal 17 amino acids of the *hph* protein, and the N-terminal 9 amino acids of the TK protein, are deleted in the bifunctional fusion protein.

B. Dominant Positive Selection of Cells Containing the HyTk Selectable Fusion Gene. To demonstrate that the HyTK selectable fusion gene encodes both *hph* and TK enzymatic activities, the frequencies with which tgCMV/HyTK conferred hygromycin resistance (Hm^{r}) (in NIH/3T3 cells and Rat-2 cells), and the ability to grow in medium containing hypoxanthine, aminopterin, and thymidine (HAT^{r}) (in Rat-2 cells), were compared with those of the parental plasmids, tgCMV/hygro and tgCMV/TK, respectively.

NIH/3T3 cells were grown in Dulbecco's Modified Eagle Medium (DMEM; Gibco Laboratories) supplemented with 10% bovine calf serum (Hyclone), 2 mM L-glutamine, 50 U/ml penicillin, and 50 µg/ml streptomycin at 37 °C in a humidified atmosphere supplemented with 10% CO₂. TK- Rat-2 cells (Topp, *Virology* 113:408, 1981) were grown in DMEM supplemented with 10% fetal bovine serum (Hyclone), 2 mM L-glutamine, 50 U/ml penicillin, and 50 µg/ml streptomycin at 37°C in a humidified atmosphere supplemented with 10% CO₂. NIH/3T3 and Rat-2 cells were transfected with the DNA vectors described above by electroporation, as follows. Exponentially growing NIH/3T3 and Rat-2 cells were harvested by trypsinization, washed free of serum, and resuspended in DMEM at a concentration of 10⁷ cells/ml. Supercoiled plasmid DNA (5 µg) was added to 800 µl of cell suspension (8x10⁶ cells), and the mixture subjected to electroporation using the Biorad Gene Pulser and Capacitance Extender (200-300 V, 960 µF, 0.4 cm electrode gap, at ambient temperature). Following transfection, the cells were returned to 9-cm dishes and grown in non-selective medium. After 24 hours, the cells were trypsinized, seeded at 6x10⁵ per 9-cm dish, and allowed to attach overnight. The non-selective medium was replaced with selective medium (containing 500 µg/ml hygromycin B for NIH3T3 cells, and 300 µg/ml hygromycin B or HAT for Rat-2 cells), and selection was continued for approximately 10-12 days until colonies were evident. The plates were stained with methylene blue and counted. The results are shown in Table 1 below. The number of colonies reported is the average number of colonies per 9-cm dish.

**TABLE 1**

| Positive Selection Using HyTK Fusion Gene | | | |
|---|---|---|---|
| | NIH/3T3 Cells | Rat-2 Cells | |
| Plasmid | No. Hm^{r} Colonies | No. Hm^{r} Colonies | No. HAT^{r} Colonies |
| tgCMV/hygro | 45 | 368 | n.t. |
| tgCMV/TK | n.t. | n.t. | 356 |
| tgCMV/HyTK | 100 | 428 | 124 |

| | | | |
|---|---|---|---|
| n.t.= not tested. | | | |

In both cell lines, tgCMV/HyTK gave rise to Hm^{r} colonies at a slightly higher frequency than tgCMV/hygro. However, in Rat-2 cells, tgCMV/HyTK was slightly less efficient than tgCMV/TK in generating HAT^{r} colonies. This demonstrates that the HyTK selectable fusion gene encodes both *hph* and TK enzymatic activities, although with altered efficiencies.

C. Negative Selection of Cells Containing the HyTK Selectable Fusion Gene. To investigate the utility of the HyTK selectable fusion gene for negative selection, the colonies resulting from each transfection (Table 1) were pooled and expanded into cell lines for further analysis. The Hm^{r} NIH/3T3 cell pools and the Hm^{r} and HAT^{r} Rat-2 cell pools were tested for GCV^{s} in a short term cell proliferation assay as follows.

The transfected NIH/3T3 and Rat-2 cells (3 x 10⁴ of each) were seeded into 9-cm tissue culture dishes in complete growth medium, and allowed to attach for 4 hours. The medium was then supplemented with various concentrations of GCV (Syntex, Palo Alto, CA), and the cells incubated for an additional 60 hours. At this time, the medium was removed, the attached cells were harvested by trypsinization and stained with trypan blue, and viable cells were counted. Cell growth was expressed as a fraction of the cell growth observed in the absence of GCV. The results shown are the average of triplicate assays.

The results shown in Figure 3 demonstrate that the HyTK selectable fusion gene confers GCV^{s} in NIH/3T3 cells. The degree of inhibition of cell growth was proportional to the concentration of GCV used, and approached 100% at a concentration of 1 µM. In contrast, NIH/3T3 cells transfected with tgCMV/hygro were not adversely affected by GCV over the range of concentrations tested (0.03 µM - 1.0 µM).

The results shown in Figure 4 indicate that the HyTK selectable fusion gene is more effective than the HSV-I TK gene for negative selection in Rat-2 cells. Growth of Rat-2 cells transfected with tgCMV/HyTK was almost completely inhibited even at the lowest concentration of GCV used (0.03 µM), whether the cells were initially selected for Hm^{r} or HAT^{r}. Growth of Rat-2 cells transfected with tgCMV/hygro was not inhibited by GCV over the range of concentrations tested (0.03 µM - 1.0 µM). The growth of Rat-2 cells transfected with tgCMV/TK was inhibited by GCV, but the concentrations required for growth inhibition were much higher than those required to inhibit the growth of Rat-2 cells transfected with tgCMV/HyTK. The Rat-2 cells transfected with tgCMV/TK were less sensitive to GCV than the Rat-2 cells transfected with tgCMV/HyTK. This appears to conflict with the result obtained when the two genes were used for positive selection in Rat-2 cells (Table 1), which indicated that the HyTK selectable fusion gene was less effective than the HSV-I TK gene in conferring HAT^{r}. A further observation concerning the relative sensitivities of these cell lines to GCV was that the NIH/3T3 cells transfected with tgCMV/HyTK were less sensitive to GCV than the Rat-2 cells transfected with tgCMV/HyTK.

D. Northern Analysis of Transfected Cell Lines. To investigate the basis for the the differential sensitivities of the Hm^{r} and HAT^{r} NIH/3T3 and Rat-2 cell pools to GCV (Figures 3 and 4), and the altered efficiency with which the HyTK selectable fusion gene gave rise to Hm^{r} and HAT^{r} colonies (Table 1), Northern blots of mRNA from each cell pool were probed with sequences from the *hph* and HSV-I TK genes, as follows.

Polyadenylated mRNA was prepared according to standard procedures (Ausabel et al., eds., *Current protocols in Molecular Biology.* Wiley, New York., 1987). RNA samples (10 µg) were electrophoresed through 1.1% agarose gels containing formaldehyde as described (Ausabel et al., *supra).* Following electrophoresis, the gels were inverted and blotted by capillary transfer in 20 x SSC onto Duralon UV nylon membranes (Stratagene). After fixing the mRNA to the membrane by UV-irradiation (0.12 J/cm²), the membranes were incubated in Stark's buffer (50% formamide, 5 x SSC, 50 mM potassium phosphate (pH 6.5), 1% SDS, 0.1% Ficoll, 0.1% PVP, 0.1% BSA, 300 µg/ml sheared and denatured salmon sperm DNA, 0.05% Sarkosyl) at 50 °C for several hours. A uniformly labelled single stranded antisense RNA probe specific for *hph* was prepared (Ausabel et al., *supra),* 1 x 10⁶ cpm/ml were added to the hybridization mixture, and the incubation was continued at 63 °C for 15 h. The membrane was then washed in 0.1 x SSC, 0.1% SDS at 63 °C, and exposed to autoradiographic film (Kodak XAR-5). For detection of HSV-I TK and β-actin sequences, gel-purified restriction fragments from the HSV-I TK and β-Actin genes were radiolabelled by random priming (Ausabel et al., *supra).* Membranes were pre-hybridized in Starks buffer at 42°C for several hours, after which 1 x 106 cpm/ml of probe was added to the hybridization mixture and incubation continued at 42°C for 15 hours. The membranes were then washed in 6 x SSC, 1% SDS at 63°C, and exposed to autoradiographic film (Kodak XAR-5).

In both Rat-2 and NIH/3T3 cells, the steady state level of mRNA detected with the *hph* probe was higher in the cells transfected with tgCMV/hygro than the cells transfected with tgCMV/HyTK and selected for Hm^{r} (Figure 5, gel A, lanes 5 and 6). This may indicate that a higher level of expression of the *hph* gene is required to confer resistance to equivalent levels of hygromycin B (300 µg/ml in Rat-2, and 500 µg/ml in NIH/3T3), due to the fact that the bifunctional fusion protein is more effective than the *hph* protein at inactivating hygromycin B, or is more stable than the *hph* protein. This conclusion is supported by the results in Table 1, which show that tgCMV/HyTK gave rise to a slightly greater number of Hm^{r} colonies in both cell lines than did tgCMV/hygro.

The RNA Northern analysis also indicated that the Rat-2 cells transfected with tgCMV/TK expressed a steady state level of mRNA similar to the Rat-2 cells transfected with tgCMV/HyTK and selected for HAT^{r} (Figure 5, gel B, lanes 2 and 4). However, tgCMV/TK gave rise to a greater number of HAT^{r} cells than did tgCMV/HyTK (Table 1). This suggests that the HyTK selectable fusion protein is less effective than the HSV-I TK protein at phosphorylating thymidine, or is less stable than the HSV-I TK protein.

Finally, the Rat-2 cells transfected with tgCMV/HyTK expressed steady state levels of mRNA several fold higher than (when selected for Hm^{r}: Figure 5, gel B, lane 3), or similar to (when selected for HAT^{r}; Figure 5, gel B, lane 4), the Rat-2 cells transfected with tgCMV/TK (Figure 5, gel B, lane 2). However, both the Rat-2 cell pools transfected with tgCMV/HyTK were over 30-fold more sensitive to GCV than the Rat-2 cells transfected with tgCMV/TK (Figure 4). This suggests that the bifunctional fusion protein is markedly more effective than the HSV-I TK protein at phosphorylating ganciclovir, or is markedly more stable than the HSV-I TK protein. The increased ability of the bifunctional fusion protein to confer GCV^{s}, and concomitant decreased ability to confer HAT^{r}, suggests that the substrate affinity of the bifunctional fusion protein is altered relative to that of the HSV-I TK protein, rather than the stability.

### Example 2

### Construction and Characterization of Retroviral Vectors Containing HyTK Selectable Fusion Gene

A. Construction of Retroviral Vectors. Two retroviral expression vectors containing the HyTK selectable fusion gene were constructed. In the first, tgLS(+)HyTK, the HyTK selectable fusion gene was placed under the regulatory control of the promoter present in the retroviral LTR. In the second, tgLS(-)CMV/HyTK, the HyTK selectable fusion gene was placed under the regulatory control of the HCMV promoter.

The retroviral expression vector tgLS(+)HyTK (the proviral structure of which is shown in Figure 2) consists of the following elements: the 5' LTR and sequences through the PstI site at nucleotide 984 of MoMSV (Van Beveren et al., *Cell 27:97,* 1981); sequences from the PstI site at nucleotide 563 to nucleotide 1040 of MoMLV (Shinnick et al., *Nature 293:543,* 1981), incorporating point mutations (ATG → TAG) which eliminate the Pr65 *gag* translation initiation codon (Bender et al., *J. Virol.* 61:1639, 1987); a fragment from tgCMV/HyTK, containing the HyTK selectable fusion gene; sequences from nucleotide 7764 and through the 3' LTR of MoMLV (Shinnick et al., *Nature 293:543,* 1981); the NruI-AlwNI fragment from pML2d (Lusky and Botchan, *Nature 293:79,* 1981), containing the bacterial replication origin; and the AlwNI-AatII fragment from pGEM1 (Promega Corporation), containing the β-lactamase gene.

The retroviral expression vector tgLS(-)CMV/HyTK (the proviral structure of which is shown in Figure 2) is similar to tgLS(+)HyTK, but carries a point mutation (AGGT → AGGC) which eliminates the MoMSV-derived splice donor sequence (transferred from the retroviral vector, ΔH [Overell et al., *Mol. Cell. Biol.* 8:1803, 1988]), and contains the HCMV promoter upstream of the HyTK selectable fusion gene sequences.

The retroviral expression vector tgLS(+)HyTK/stop (the proviral structure of which is shown in Figure 2) was derived from tgLS(+)HyTK by inserting the universal translation terminator oligonucleotide (Pharmacia), 5'-GCTTAATTAATTAAGC-3', at the NaeI site located near the junction of the *hph* and HSV-I TK sequences of the HyTK selectable fusion gene.

B. Generation of Stable Cell Lines Producing Retroviral Vectors. Stable Ψ2 packaging cell lines were generated which produce the above ecotropic retroviruses as follows. Ψ2 cells (Mann et al., *Cell* 33:153, 1983) were grown in Dulbecco's Modified Eagle Medium (DMEM; Gibco Laboratories) supplemented with 10% bovine calf serum (Hyclone), 2 mM L-glutamine, 50 U/ml penicillin, and 50 µg/ml streptomycin at 37 °C in a humidified atmosphere supplemented with 10% CO₂. PA317 cells (Miller and Buttimore, *Mol. Cell. Biol. 6*:2895, 1986) were grown in DMEM supplemented with 10% fetal bovine serum (Hyclone), 2 mM L-glutamine, 50 U/ml penicillin, and 50 µg/ml streptomycin at 37 °C in a humidified atmosphere supplemented with 10% CO₂.

The retroviral expression vectors described above were first transfected into PA317 amphotropic packaging cells by electroporation. Amphotropic virions produced by the transiently transfected PA317 packaging cells were then used to infect the Ψ2 cells as follows. Exponentially growing PA317 cells were harvested by trypsinization, washed free of serum, and resuspended in DMEM at a concentration of 10⁷ cells/ml. Supercoiled plasmid DNA (5 µg) was added to 800 µl of cell suspension (8x10⁶ cells), and the mixture subjected to electroporation using the Biorad Gene Pulser and Capacitance Extender (200-300 V, 960 µF, 0.4 cm electrode gap, at ambient temperature). The transfected PA317 cells were then transferred to a 9-cm tissue culture dish containing 10 ml of complete growth medium supplemented with 10 mM sodium butyrate (Sigma Chemical Co.), and allowed to attach overnight. After 15 hours, the medium was removed and replaced with fresh medium. After a further 24 hours, the medium containing transiently produced amphotropic retrovirus particles was harvested, centrifuged at 2000 rpm for 10 min, and used to infect the Ψ2 ecotropic packaging cells. Exponentially dividing Ψ2 cells were plated at a density of 10⁶ cells per 9-cm tissue culture dish, and allowed to attach overnight. The following day, the medium was removed and replaced with serial dilutions of the virus-containing supernatant (6 ml/dish) in medium supplemented with 4 µg/ml polybrene (Sigma Chemical Co.). Infection of the Ψ2 cells by the viral particles was allowed to proceed overnight, and then the supernatant was replaced with complete growth medium. Infected cells were selected for drug resistance after a further 8-24 hours of growth by adding hygromycin B (Calbiochem) to a final concentration of 500 µg/ml. Colonies of Hm^{r} cells were isolated using cloning cylinders 12-14 days later, and individually expanded into bulk cultures for analysis. Southern analysis (data not shown) revealed that the proviral structures were intact in six out of six independent clones, indicating that the HyTK selectable fusion gene is compatible with the retroviral life cycle.

C. Transduction of Hm^{r}, HAT^{r}, and GCV^{s} by tgLS(+)HyTK and tgLS(-) CMV/HyTK Retroviral Expression Vectors. The infected Ψ2 clones were titered on NIH/3T3 cells (selecting for Hm^{r}), and on Rat-2 cells (selecting for Hm^{r}, or for HAT^{r}) (Table 2), as follows. The Ψ2 clones producing the virus were grown to confluence in 9-cm tissue culture dishes, then fed with 15 ml of drug-free medium. After an overnight incubation, aliquots of supernatant were taken for assay. Exponentially dividing NIH/3T3 or Rat-2 cells were harvested by trypsinization and seeded at a density of 2.5 x 10⁴ cells per 35 mm well in 6-well tissue culture trays. The following day, the medium was replaced with serial dilutions of virus-containing supernatant (1 ml/well) in medium supplemented with 4 µg/ml polybrene (Sigma Chemical Co.). All supernatants were centrifuged at 2000 rpm for 10 min before use to remove viable cells. Infection was allowed to proceed overnight, and then the supernatant was replaced with complete growth medium. Infected cells were selected for drug resistance after a further 8-24 hours of growth by adding hygromycin B (Calbiochem) to a final concentration of 500 µg/ml (NIH/3T3 cells) or 300 µg/ml (Rat-2 cells), or by adding HAT supplement (Gibco) (Rat-2 cells). After a total of 12-14 days of growth, cells were fixed *in situ* with 100% methanol, and stained with methylene blue.

As shown in Table 2, below, both retroviruses conferred Hm^{r} (to NIH/3T3 and Rat-2 cells) and HAT^{r} (to Rat-2 cells). All viruses were harvested from a clone of infected Ψ2 cells.

**TABLE 2**

| Titers of ecotropic retroviruses produced by Ψ2 packaging cells on NIH/3T3 cells and Rat-2 cells. | | | |
|---|---|---|---|
| | NIH/3T3 | Rat-2 | |
| Virus | Hm^{r} CFU/ml | Hm^{r} CFU/ml | HAT^{r} CFU/ml |
| | | | |
| tgLS(+)HyTK clone 5.5 | 1.8 x 10⁷ | 1.6 x 10⁷ | 4 x 10⁶ |
| | | | |
| tgLS(-)CMV/HyTK clone 6.2 | 1 x 10⁶ | 1 x 10⁶ | 8 x 10⁵ |
| | | | |

To demonstrate that the tgLS(+)HyTK and tgLS(-)CMV/HyTK retroviruses also conferred GCV^{s}, NIH/3T3 cells infected with the two retroviruses, were selected for Hm^{r} (500 µg/ml) for 10 days, and then pooled, expanded, and tested for GCV^{s} in the following long-term cell proliferation assay.

Uninfected NIH/3T3 cells and the infected NIH/3T3 cell pools were plated at relatively low cell density (10⁴ cells/9-cm dish) in complete growth medium and allowed to attach for 4 hours. The medium was then supplemented with hygromycin B (500 µg/ml), with or without 1 µM GCV, and the cells incubated for a period of 10 days. The medium was then removed and the cells were fixed *in situ* with 100% methanol and stained with methylene blue. The growth of both cell pools, as measured by colony formation, was almost completely inhibited by GCV (Figure 6, plates e and g), indicating that both retroviruses conferred Hm^{r} and GCV^{s}. Uninfected NIH/3T3 were resistant to this concentration of GCV, and grew to a confluent monolayer (Figure 6, plate b), but were completely killed by 500 µg/ml Hm (Figure 6, plate c). Colonies of cells resistant to both Hm and GCV were obtained at a low frequency (10⁻⁴-10⁻³) from the retrovirus-infected populations (Figure 6, plates e and g). The proviruses present in the cells that gave rise to these colonies had likely suffered point mutations, or very small deletions or rearrangements in the HSV-I TK moiety which eliminated the ability to phosphorylate GCV. Similar results were also obtained with Rat-2 cell lines infected with tgLS(+)HyTK or with tgLS(-)CMV/HyTK (data not shown).

### Example 3

### Evidence for the Production of a Bifunctional Selectable Fusion Protein

In HSV-I infected cells, the HSV-I TK gene normally utilizes three translation initiation sites, and encodes three nested polypeptides which all possess TK activity (Haarr et al., *J. Virol. 56:512,* 1985). Since the HyTK selectable fusion gene retains two of these initiation codons, it was conceivable that, as a result of translation initiation at one or both of these internal AUG codons, the HyTK selectable fusion gene might also encode nested polypeptides possessing TK activity. The bifunctional fusion protein, while retaining *hph* activity, might or might not possess TK activity. To rule out this possibility, an oligonucleotide sequence, 5'-GCTTAATTAATTAAGC-3', bearing translation termination codons in all three reading frames was introduced into the HyTK selectable fusion gene in tgLS(+)HyTK, generating the construct designated tgLS(+)HyTK/stop (Figure 1B). The oligonucleotide was inserted at a NaeI site downstream of the *hph*-derived sequences, but upstream of the two internal AUG codons in the HSV-I TK-derived sequences of the HyTK selectable fusion gene (Figure 1B). The tgLS(+)HyTK and tgLS(+)HyTK/stop retroviral expression vectors were transfected into Ψ2 cells, and the transiently produced virus was used to infect Rat-2 cells, which were then selected for Hm^{r} or HAT^{r} (Table 3). The retroviral expression vector tgLS(+)HyTK transduced both Hm^{r} and HAT^{r}, but retroviral expression vector tgLS(+)HyTK/stop was only able to transduce Hm^{r}. Insertion of the translation termination codons completely abolished the ability of the retrovirus to transduce HAT^{r}, indicating that the internal translation initiation codons are not utilized in the HyTK selectable fusion gene, and the HyTK selectable fusion gene does indeed encode a bifunctional fusion protein. Viruses were harvested from transiently transfected Ψ2 cells.

**TABLE 3**

| Titers of ecotropic retroviruses produced transiently in Ψ2 packaging cells on NIH/3T3 cells and Rat-2 cells. | | | |
|---|---|---|---|
| | NIH/3T3 | Rat-2 | |
| Virus | Hm^{r} CFU/ml | Hm^{r} CFU/ml | HAT^{r} CFU/ml |
| tgLS(+)HyTK | 4.5 x 10⁴ | 9.5 x 10³ | 1.1 x 10⁴ |
| tgLS(-)CMV/HyTK | 2.6 x 10⁴ | 5.9 x 10⁴ | 0 |

As described in the above examples, retroviral expression vectors containing the HyTK selectable fusion gene were constructed and used to demonstrate the efficacy of the HyTK selectable fusion gene for positive and negative selection in NIH/3T3 and Rat-2 cells. High titer virus stocks were generated, which conferred both Hm^{r} and HAT^{r} on infected cells. Infected cells contained unrearranged proviruses and were killed (>99.9%) by GCV. The HyTK selectable fusion gene was slightly more effective than the *hph* gene at conferring Hm^{r} in both NIH/3T3 and Rat-2 cells (Table 1). Genetic evidence that the HyTK selectable fusion gene encodes a bifunctional fusion protein possessing *hph* and HSV-I TK enzymatic activities was obtained by inserting translation termination codons into the HyTK selectable fusion gene (in tgLS(+)HyTK/stop; Figure 2), downstream of the *hph*-derived sequences, but upstream of the HSV-I TK-derived sequences. As would be expected if the HyTK selectable fusion gene encoded a bifunctional fusion protein, insertion of the translation termination codons left the ability of the virus to confer Hm^{r} intact, but abolished the ability of the retrovirus to transduce HAT^{r} (Table 3). When compared with the HSV-I TK gene in Rat-2 cells, the HyTK selectable fusion gene was slightly less effective at conferring ability to grow in HAT medium (Table 1), but markedly more effective at conferring GCV^{s} (Figure 4). These observations cannot be explained on the basis of the relative steady state levels of mRNA expression (Figure 5), nor on the basis of changes in the stability of the HyTK selectable fusion protein. The apparent contradiction might be explained by hypothesizing that the HSV-I TK-derived moiety of the HyTK selectable fusion protein possesses a substrate affinity different from that of the wild-type HSV-I TK protein (possibly due to conformational change), with a reduced ability to phosphorylate thymidine and an increased ability to phosphorylate GCV. Altered substrate affinities have been noted previously in a number of pathogenic drug-resistant strains of HSV-I, which encode mutant TK proteins that exhibit a reduced ability to phosphorylate thymidine analogs, yet retain the ability to phosphorylate thymidine (Larder et al., *J. Biol. Chem. 258*:2027, 1983; Palu et al., *Virus Res. 13*:303, 1989; Larder and Darby, *Antiviral Res. 4*:1, 1984). The slightly increased efficiency with which the HyTK selectable fusion gene confers Hm^{r}, relative to the *hph* gene (Table 1), may be due to an increase in protein stability, or an increased specific activity of the phosphotransferase.

Moreover, a single approximately 76 kD protein was specifically immunoprecipitated by a rabbit polyclonal antiserum directed against HSV-I TK from extracts of cells expressing the HyTK selectable fusion gene. Thus the phenotype conferred by the HyTK selectable fusion gene was not due to internal translation initiation in the HSV-I TK derived moiety of the gene, and the HyTK selectable fusion gene does indeed encode a bifunctional selectable fusion gene.

## Claims

1. A selectable fusion gene comprising two genes, a first gene being a dominant positive selectable gene which is fused to, and in reading frame with, a second gene which is a negative selectable gene, wherein the selectable fusion gene encodes a single bifunctional fusion protein which is capable of conferring both a dominant positive selectable phenotype and a negative selectable phenotype on a cellular host.

2. A selectable fusion gene according to claim 1 wherein the dominant positive selectable gene is *hph, neo* or *gpt* and the negative selectable gene is HSV-1 TK, HPRT, APRT or *gpt.*

3. A selectable fusion gene according to claim 1 wherein the dominant positive selectable marker is *hph* and the negative selectable marker is HSV-I TK.

4. A selectable fusion gene according to claim 3 encoding the sequence of amino acids 1-691 of SEQ ID NO:2.

5. A selectable fusion gene according to claim 4 comprising the sequence of nucleotides 1-2073 of SEQ ID NO:1.

6. A recombinant expression vector comprising a selectable fusion gene according to any of claims 1 to 5.

7. A recombinant expression vector according to claim 6 which is a retrovirus.

8. A cell transduced with a recombinant expression vector according to claim 6 or 7.

9. A method of conferring a dominant positive and negative selectable phenotype on a cell, the method comprising transducing the cell with a recombinant expression vector according to claim 6 or 7.

10. A method of isolating cells having a negative selectable phenotype, the method comprising:
(a) transducing a population of cells with a recombinant expression vector having a selectable fusion gene comprising two genes, a first gene being a dominant positive selectable gene which is fused to, and in reading frame with, a second gene which is a negative selectable gene, thereby conferring the cells with a dominant positive selectable phenotype and a negative selectable phenotype;
(b) applying positive selection to select cells having a dominant positive selectable phenotype, thereby concomitantly selecting cells having a negative selectable phenotype.

## Patentansprüche

1. Selektionsfusionsgen, das zwei Gene umfaßt, wobei das erste ein dominant-positives Selektionsgen ist, das im Leserahmen mit einem zweiten Gen fusioniert ist, das ein negatives Selektionsgen ist, wobei das Selektionsfusionsgen ein einzelnes bifunktionelles Fusionsprotein codiert, das einen zellulären Wirt sowohl mit einem dominant-positiven Selektionsphänotyp als auch mit einem negativen Selektionsphänotyp ausstatten kann.

2. Selektionsfusionsgen nach Anspruch 1, wobei das dominant-positive Selektionsgen hph, neo oder gpt ist und das negative Selektionsgen HSV-I-TK, HPRT, APRT oder gpt ist.

3. Selektionsfusionsgen nach Anspruch 1, wobei der dominant-positive Selektionsmarker hph ist und der negative Selektionsmarker HSV-I-TK ist.

4. Selektionsfusionsgen nach Anspruch 3, das die Sequenz der Aminosäuren 1-691 von Seq ID No: 2 codiert.

5. Selektionsfusionsgen nach Anspruch 4, das die Sequenz der Nucleotide 1-2073 von SEQ ID No: 1 umfaßt.

6. Rekombinanter Expressionsvektor, der ein Selektionsfusionsgen nach einem der Ansprüche 1 bis 5 umfaßt.

7. Rekombinanter Expressionsvektor nach Anspruch 6, der ein Retrovirus ist.

8. Zelle, die mit einem rekombinanten Expressionsvektor nach Anspruch 6 oder 7 transduziert ist.

9. Verfahren zur Ausstattung einer Zelle mit einem dominant-positiven und negativen Selektionsphänotyp, das die Transduktion der zelle mit einem rekombinanten Expressionsvektor nach Anspruch 6 oder 7 umfaßt.

10. Verfahren zur Isolierung von Zellen mit einem negativen Selektionsphänotyp, das umfaßt:
(a) Transduktion einer Zellpopulation mit einem rekombinanten Expressionsvektor mit einem Selektionsfusionsgen, das zwei Gene umfaßt, wobei ein erstes Gen ein dominant-positives Selektionsgen ist, das im Leserahmen mit einem zweiten Gen fusioniert ist, das ein negatives Selektionsgen ist, wobei die Zellen mit einem dominant-positiven Selektionsphänotyp und einem negativen Selektionsphänotyp ausgestattet werden;
(b) positive Selektion zur Auswahl von Zellen mit einem dominant-positiven Selektionsphänotyp, wobei gleichzeitig Zellen selektiert werden, die einen negativen Selektionsphänotyp haben.

## Revendications

1. Gène de fusion sélectionnable comprenant deux gènes, un premier gène qui est un gène dominant sélectionnable de façon positive fusionné à, et dans un cadre de lecture avec, un second gène qui est un gène sélectionnable de façon négative, le gène de fusion sélectionnable codant pour une unique protéine de fusion bi-fonctionnelle qui est capable de conférer à la fois un phénotype dominant sélectionnable de façon positive et un phénotype sélectionnable de façon négative à un hôte cellulaire.

2. Gène de fusion sélectionnable selon la revendication 1 dans lequel le gène dominant sélectionnable de façon positive est *hph, neo* ou *gpt* et le gène sélectionnable de façon négative est HSV-1 TK, HPRT, APRT ou *gpt.*

3. Gène de fusion sélectionnable selon la revendication 1 dans lequel le marqueur dominant sélectionnable de façon positive est *hph* et le marqueur sélectionnable de façon négative est HSV-I TK.

4. Gène de fusion sélectionnable selon la revendication 3 codant pour la séquence d'acides aminés 1-691 de SEQ ID NO:2.

5. Gène de fusion sélectionnable selon la revendication 4 comprenant la séquence de nucléotides 1-2073 de SEQ ID NO:1.

6. Vecteur d'expression recombinant comprenant un gène de fusion sélectionnable selon l'une quelconque des revendications 1 à 5.

7. Vecteur d'expression recombinant selon la revendication 6 qui est un rétrovirus.

8. Cellule transduite avec un vecteur d'expression recombinant selon la revendication 6 ou 7.

9. Méthode pour conférer un phénotype dominant sélectionnable de façon négative et de façon positive à une cellule, la méthode comprenant l'étape de transduction de la cellule avec un vecteur d'expression recombinant selon la revendication 6 ou 7.

10. Méthode d'isolement de cellules ayant un phénotype sélectionnable de façon négative, la méthode comprenant les étapes consistant :
(a) à transduire une population de cellules avec un vecteur d'expression recombinant ayant un gène de fusion sélectionnable comprenant deux gènes, un premier gène qui est un gène dominant sélectionnable de façon positive fusionné à, et dans un cadre de lecture avec, un second gène qui est un gène sélectionnable de façon négative, conférant ainsi aux cellules un phénotype dominant sélectionnable de façon positive et un phénotype sélectionnable de façon négative;
(b) à appliquer une sélection positive pour sélectionner des cellules ayant un phénotype dominant sélectionnable de façon positive, sélectionnant ainsi de façon concomitante des cellules ayant un phénotype sélectionnable de façon négative.
